(19) Europäisches Patentamt
European Patent Office
Office européen des brevets

(11) EP 4 223 512 A1

(12) **EUROPEAN PATENT APPLICATION**
published in accordance with Art. 153(4) EPC

(43) Date of publication:
**09.08.2023 Bulletin 2023/32**

(21) Application number: **21875250.9**

(22) Date of filing: **16.09.2021**

(51) International Patent Classification (IPC):
***B32B 27/00*** *(2006.01)* ***B32B 5/18*** *(2006.01)*
***F24F 3/14*** *(2006.01)* ***F24F 7/08*** *(2006.01)*
***F28F 3/00*** *(2006.01)*

(52) Cooperative Patent Classification (CPC):
**B32B 5/18; B32B 27/00; F24F 3/14; F24F 7/08; F28F 3/00;** Y02B 30/56

(86) International application number:
**PCT/JP2021/034104**

(87) International publication number:
**WO 2022/070948 (07.04.2022 Gazette 2022/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2020 JP 2020165951**

(71) Applicants:
- **Daicel Corporation**
  **Osaka 530-0011 (JP)**
- **Daikin Industries, Ltd.**
  **Osaka-shi, Osaka 530-8323 (JP)**

(72) Inventors:
- **NISHIO, Naotaka**
  **Tokyo 108-8230 (JP)**
- **IWAYAMA, Masashi**
  **Tokyo 108-8230 (JP)**
- **IWAYAMA, Megumi**
  **Tokyo 108-8230 (JP)**
- **NAKAZAWA, Takema**
  **Osaka-shi, Osaka 530-8323 (JP)**

(74) Representative: **Grünecker Patent- und Rechtsanwälte**
**PartG mbB**
**Leopoldstraße 4**
**80802 München (DE)**

(54) **LAMINATE**

(57) Provided is a laminate having low air permeability and excellent moisture permeability. A laminate 1 is provided with a porous substrate 11 and a moisture-permeable membrane 12 disposed on at least one side 11a of the porous substrate 11, the moisture-permeable membrane 12 being formed of a resin having an attraction value relative to water of -110 kcal/mol or less and a repulsive value relative to water of 35 kcal/mol or greater, the attraction value relative to water being a negative integral value while the repulsive value relative to water being a positive integral value in an energy histogram of a combination of solute and solvent obtained from a process of calculating free energy based on energy representation.

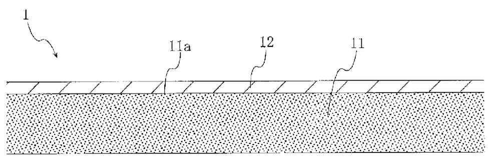


FIG. 1

EP 4 223 512 A1

## Description

### Technical Field

The present disclosure relates to a laminate. More specifically, the present disclosure relates to a laminate provided with a porous substrate and a moisture-permeable membrane disposed on at least one side of the porous substrate. The present application claims priority from the Japanese patent application No. 2020-165951 filed in Japan on September 30, 2020, the content of which is incorporated herein by reference.

### Background Art

Heat exchange ventilation devices that exchange heat between supply air and exhaust air during ventilation are known as devices capable of ventilating without impairing the effects of cooling and heating.

Heat exchange sheets for performing heat exchange are used in heat exchange ventilation devices. As a partition member that physically separates supply air and exhaust air, a heat exchange sheet is required to have a low air permeability (gas barrier property), for preventing supply air and exhaust air from mixing with each other, and heat transfer property, for exchanging heat between supply air and exhaust air. In addition, a heat exchange sheet (total heat exchange sheet) used in a total heat exchanger, which exchanges temperature (sensible heat) and moisture (latent heat) between supply air and exhaust air, is further required to have a high moisture permeability.

A sheet using a moisture-permeable membrane formed of, for example, calcium chloride or lithium chloride which have deliquescency, or a low molecular weight compound such as sulfuric acid or sodium hydroxide can be considered as an example of a heat exchange sheet used in a total heat exchanger. Among them, a moisture-permeable membrane formed of calcium chloride or lithium chloride is widely used from the perspective of safety. However, a moisture-permeable membrane formed of a compound having deliquescency or a low molecular weight compound is highly soluble in water and has poor water resistance.

Another known heat exchange sheet used in a total heat exchanger is a partition member for total heat exchange sheet having a porous substrate and a hydrophilic polymer compound provided on the surface and the inner part of the porous substrate, the hydrophilic polymer compound being a polymer of a compound having a quaternary ammonium group and an amide group (see Patent Document 1).

### Citation List

#### Patent Document

Patent Document 1: JP 2014-55683 A

### Summary of Invention

#### Technical Problem

However, the hydrophilic polymer compound using a polymer of a compound having a quaternary ammonium group and an amide group described in Patent Document 1 has insufficient moisture permeability. In particular, the moisture permeability in a low temperature and low humidity environment is insufficient.

Accordingly, an object of the present disclosure is to provide a laminate having low air permeability and excellent moisture permeability.

#### Solution to Problem

As a result of diligent studies to achieve the above object, the inventors of the present disclosure found that a laminate provided with a porous substrate and a moisture-permeable membrane that is disposed on at least one side of the porous substrate and that is formed of a specific resin has low air permeability and excellent moisture permeability. The present invention relates to what has been completed based on these findings.

The present disclosure provides a laminate provided with a porous substrate and a moisture-permeable membrane disposed on at least one side of the porous substrate,

the moisture-permeable membrane being formed of a resin having an attraction value relative to water of -110 kcal/mol or less and a repulsive value relative to water of 35 kcal/mol or greater, the attraction value relative to water being a negative integral value while the repulsive value relative to water being a positive integral value in an energy histogram of a combination of solute and solvent obtained from a process of calculating free energy based on energy representation.

The resin described above preferably includes (i) a hydrophilic portion, and (ii) one or more hydrophobic portions, the hydrophilic portion being a group represented by Formula (A) and/or a sulfone group, and the one or more hydrophobic portions being selected from the group consisting of: a monovalent hydrocarbon group having two or more carbons in which one or more hydrogen atoms may be substituted by a fluorine atom; a polystyrene backbone; a polyolefin backbone in which one or more hydrogen atoms may be substituted with a fluorine atom; a polyalkadiene backbone; a polyphenylene backbone; a cellulose backbone; and a polyglycerin backbone;

[Chem. 1]

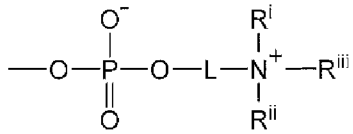

(A)

where in Formula (A), $R^i$, $R^{ii}$, and $R^{iii}$ are the same or different and each represents an alkyl group having from 1 to 4 carbons, and L represents a divalent linear hydrocarbon group having from 1 to 4 carbons.

The sulfone group is preferably included in the resin as one or more groups selected from the group consisting of: a constituent unit derived from a styrenesulfonic acid; a constituent unit derived from vinylsulfonic acid; an aromatic sulfonic acid group; and a sulfonic acid group bonded to a primary carbon.

The resin is preferably one or more resins selected from the group consisting of: a resin having a sulfone group and a polyolefin backbone in which one or more hydrogen atoms may be substituted with a fluorine atom; a resin having a sulfone group and a polyphenylene backbone; a resin having a sulfone group and a polystyrene backbone; a resin having a sulfone group and a polyalkadiene backbone; a resin having a group represented by Formula (A) above and a polyolefin backbone; and a resin having a group represented by Formula (A) above and a monovalent hydrocarbon group having two or more carbons in which one or more hydrogen atoms may be substituted with a fluorine atom.

The present disclosure also provides a method of calculating an attraction value and a repulsive value relative to a solvent of a target molecule, the method including:

a solvation system creation step of arranging the target molecule in a cubic imaginary space and arranging the solvent molecules around the target molecule in the imaginary space;
a molecular dynamics calculation step of establishing an equilibrium state of the solvation system at room temperature and normal pressure;
a histogram creation step of creating, in the imaginary space, an energy histogram of a combination of solute and solvent in the equilibrium state by free energy calculation based on energy representation; and
an integration step of calculating a negative integral value in the obtained energy histogram as the attraction value relative to solvent of the target molecule and calculating a positive integral value in the obtained energy histogram as the attraction value relative to solvent of the target molecule.

The molecular dynamics calculation step preferably includes:

a first calculation phase, in which a molecular dynamics calculation is performed at a temperature of over 300 K, a pressure of 1 MPa or greater, and with an addition of an energy constraint of 1 kcal/(mol·Å$^2$) or greater to a minimum energy structure;
a second calculation phase after the first calculation phase, in which a molecular dynamics calculation is performed while the addition of the energy constraint is reduced until the addition reaches 0 kcal/(mol·Å$^2$);
a third calculation phase after the second calculation phase, in which a molecular dynamics calculation is performed while the pressure is reduced until the pressure reaches 1 atm;
a fourth calculation phase after the third calculation phase, in which a molecular dynamics calculation is performed while the temperature is reduced until the temperature reaches 300 K; and
a fifth calculation phase after the fourth calculation phase, in which a molecular dynamics calculation is performed at a temperature of 300 K, a pressure of 1 atm, and with the energy constraint lifted, resulting in an equilibrium state of the solvation system at room temperature and normal pressure.

The target molecule is preferably a molecule having a hydrophilic portion and a hydrophobic portion, and the solvent is preferably water.

The present disclosure also provides a calculation device for calculating an attraction value and a repulsive value relative to a solvent of a target molecule, the device including:

a solvation system creation portion that arranges the target molecule in a cubic imaginary space and arranges the solvent molecules around the target molecule in the imaginary space;
a molecular dynamics calculation portion that establishes an equilibrium state of the solvation system at room temperature and normal pressure;
a histogram creation portion that creates, in the imaginary space, an energy histogram of a combination of solute and solvent in the equilibrium state by free energy calculation based on energy representation; and
an integration portion that calculates a negative integral value in the obtained energy histogram as the attraction value relative to solvent of the target molecule and calculates a positive integral value in the obtained energy histogram as the attraction value relative to solvent of the target molecule.

## Advantageous Effects of Invention

The laminate of the present disclosure has low air permeability and excellent moisture permeability. The laminate of the present disclosure also has excellent moisture permeability in a low temperature and low humidity environment. As such, the laminate of the present disclosure can be particularly preferably used as a total heat exchange sheet.

## Brief Description of Drawings

FIG. 1 is a schematic cross-sectional view illustrating a laminate of an embodiment of the present disclosure.
FIG. 2 is a flowchart illustrating a calculation method of an embodiment of the present disclosure.

## Description of Embodiments

### Laminate

The laminate according to an embodiment of the present disclosure is provided with at least a porous substrate and a moisture-permeable membrane disposed on at least one side of the porous substrate. The moisture-permeable membrane may be disposed on one side of the porous substrate, or may be disposed on both sides of the porous substrate.
FIG. 1 is a schematic cross-sectional view illustrating a laminate of an embodiment of the present disclosure. A laminate 1 is provided with a porous substrate 11 and a moisture-permeable membrane 12 disposed on one side 11a of the porous substrate 11.
The moisture-permeable membrane is formed of a resin having an attraction value relative to water of -110 kcal/mol or less and a repulsive value relative to water of 35 kcal/mol or greater, the attraction value relative to water being a negative integral value while the repulsive value relative to water being a positive integral value in an energy histogram of a combination of solute and solvent obtained from a process of calculating free energy based on energy representation. Note that in the present specification, a resin having the attraction value and the repulsive value relative to water may be referred to as a "specific resin". Only one type of the specific resin may be used, or two or more types of the specific resin may be used.
In the specific resin described above, the hydrophilic portion and the hydrophobic portion are present in a well-balanced manner. As such, it is inferred that a structure in which the hydrophilic portion and the hydrophobic portion are phase-separated is formed in the moisture-permeable membrane formed of the specific resin, and that the hydrophilic portion can function as a water-guiding path to allow more water vapor to pass through, resulting in excellent moisture permeability. Further, in general, a moisture-permeable membrane formed of calcium chloride or potassium chloride tends to have an extremely low moisture absorption amount and inferior moisture permeability in a low temperature and low humidity environment; however, regarding the moisture-permeable membrane formed of the specific resin described above, the moisture absorption amount does not become extremely low while the moisture permeability is excellent even in a low temperature and low humidity environment. In addition, when the repulsive value is increased, it is also possible to obtain a moisture-permeable membrane that has excellent moisture permeability, is difficult to dissolve in water, and has excellent water resistance. Furthermore, since the pH of the aqueous solution of the specific resin described above is weakly acidic, the resulting moisture-permeable membrane has an excellent metal corrosion resistance compared to that of a moisture-permeable membrane using a known resin that is strongly acidic and that has an acidic functional group such as a sulfonyl group.
The energy representation above is described in, for example, N. Matubayasi et al., J. Chem. Phys. 113, 6070-6081 (2000). The energy histogram of a combination of solute and solvent obtained from a process of calculating free energy based on energy representation can be created using free-energy calculation software such as ERmod 0.3.5.
More specifically, the attraction value and repulsive value relative to water of the specific resin can be calculated

using a method of calculating an attraction value and a repulsive value described below with water set as the solvent.

The attraction value relative to water of the specific resin is -110 kcal/mol or less, preferably -130 kcal/mol or less, more preferably -180 kcal/mol or less, even more preferably -200 kcal/mol or less, and particularly preferably -250 kcal/mol or less. The lower the attraction value, the higher the hydrophilicity tends to be. From the viewpoint of achieving superior water resistance, the attraction value relative to water of the specific resin is, for example, -600 kcal/mol or greater, and is preferably -500 kcal/mol or greater.

The repulsive value relative to water of the specific resin is 35 kcal/mol or greater, preferably 50 kcal/mol or greater, more preferably 80 kcal/mol or greater, even more preferably 100 kcal/mol or greater, and particularly preferably 140 kcal/mol or greater. The higher the repulsive value, the higher the hydrophobicity tends to be. From the viewpoint of achieving superior moisture permeability, the repulsive value relative to water of the specific resin is, for example, 300 kcal/mol or less, and is preferably 200 kcal/mol or less.

The specific resin described above preferably includes (i) a hydrophilic portion that is a group represented by Formula (A) below and/or a sulfone group, and (ii) one or more hydrophobic portions selected from the group consisting of a monovalent hydrocarbon group having two or more carbons in which one or more hydrogen atoms may be substituted by a fluorine atom, a polystyrene backbone, a polyolefin backbone in which one or more hydrogen atoms may be substituted with a fluorine atom, a polyalkadiene backbone, a polyphenylene backbone, a cellulose backbone, and a polyglycerin backbone. A resin having such hydrophilic portion and such hydrophobic portion tends to have the attraction value and the repulsive value relative to water. Furthermore, when a resin having the attraction value and repulsive value relative to water and having the hydrophilic portion and the hydrophobic portion is used to form a moisture-permeable membrane, the resulting moisture-permeable membrane has a low air permeability and excellent moisture permeability, and tends have excellent moisture permeability in a low temperature and low humidity environment. Note that a resin does not necessarily have an attraction value and repulsive value relative to water in the above-described values just because the resin includes the hydrophilic portion and the hydrophobic portion; instead, the values of the attraction value and repulsive value relative to water are determined by the structure of the overall resin molecule. The specific resin may include only one type of each of the hydrophilic portion and the hydrophobic portion, or may include two or more types of each of the hydrophilic portion and the hydrophobic portion.

[Chem. 1]

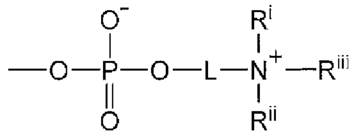

(A)

In Formula (A), $R^i$, $R^{ii}$, and $R^{iii}$ are the same or different and each represents an alkyl group having from 1 to 4 carbons. Examples of the alkyl group having from 1 to 4 carbons include a methyl group, an ethyl group, a propyl group, a butyl group, and a t-butyl group. Among these, a methyl group is preferred.

In Formula (A), L represents a divalent linear hydrocarbon group having from 1 to 4 carbons, and examples thereof include an alkylene group, an alkenylene group, and an alkynylene group. Examples of the alkylene group include a methylene group, a dimethylene group, a trimethylene group, and a tetramethylene group. Examples of the alkenylene group include an ethynylene group, a 1-propenylene group, and a 1-butenylene group. Among these, the divalent linear hydrocarbon group is preferably an alkylene group, and more preferably a dimethylene group.

Examples of the group represented by Formula (A) above include a phosphorylcholine group.

The sulfone group is preferably included in the specific resin as a constituent unit derived from a styrenesulfonic acid, a constituent unit derived from vinylsulfonic acid, an aromatic sulfonic acid group, or a sulfonic acid group bonded to a primary carbon. The aromatic sulfonic acid group is a group having a sulfonic acid group on a carbon atom constituting an aromatic group. Only one type of the sulfone group may be included, or two or more types of the sulfone group may be included.

In the monovalent hydrocarbon group having two or more carbons in which one or more hydrogen atoms may be substituted by a fluorine atom, examples of a monovalent hydrocarbon group having two or more carbons include a monovalent aliphatic hydrocarbon group, a monovalent alicyclic hydrocarbon group, a monovalent aromatic hydrocarbon group, and a monovalent group in which two or more of the groups listed above are bonded.

Examples of the monovalent aliphatic hydrocarbon group include an alkyl group, an alkenyl group, and an alkynyl group. Examples of the alkyl group include a linear or branched alkyl group, such as an ethyl group, a propyl group, an isopropyl group, a butyl group, a hexyl group, an octyl group, an isooctyl group, a decyl group, a dodecyl group, and a stearyl group. Examples of the alkenyl group include a linear or branched alkenyl group, such as a vinyl group, an allyl group, a methallyl group, a 1-propenyl group, an isopropenyl group, a 1-butenyl group, a 2-butenyl group,

a 3-butenyl group, a 1-pentenyl group, a 2-pentenyl group, a 3-pentenyl group, a 4-pentenyl group, and a 5-hexenyl group. Examples of the alkynyl group include a linear or branched alkynyl group, such as an ethynyl group and a propynyl group.

Examples of the monovalent alicyclic hydrocarbon group include: a $C_{3-12}$ cycloalkyl group, such as a cyclopropyl group, a cyclobutyl group, a cyclopentyl group, a cyclohexyl group, and a cyclododecyl group; a $C_{3-12}$ cycloalkenyl group, such as a cyclohexenyl group; and a $C_{4-15}$ crosslinked cyclic hydrocarbon group, such as a bicycloheptanyl group and a bicycloheptenyl group.

Examples of the monovalent aromatic hydrocarbon group include a $C_{6-14}$ aryl group (in particular, a $C_{6-10}$ aryl group), such as a phenyl group and a naphthyl group.

Among these, the monovalent hydrocarbon group having two or more carbons is preferably a monovalent aliphatic hydrocarbon group, more preferably a linear or branched alkyl group, and further preferably a linear alkyl group.

From the viewpoint of a more appropriate repulsive value, the number of carbons of the monovalent hydrocarbon group having two or more carbons is preferably from 4 to 26, more preferably from 8 to 22, and even more preferably from 10 to 20.

When the monovalent hydrocarbon group having two or more carbons in which one or more hydrogen atoms may be substituted by a fluorine atom is a monovalent fluorine-substituted hydrocarbon group, the monovalent fluorine-substituted hydrocarbon group is preferably a perfluorohydrocarbon group in which all hydrogen atoms are substituted with a fluorine atom.

Examples of an olefin constituting the polyolefin backbone in the polyolefin backbone in which one or more hydrogen atoms may be substituted with a fluorine atom include $\alpha$-olefins such as ethylene, propylene, 1-butene, and 4-methyl-1-pentene. The olefin constituting the polyolefin backbone may be of one type, or may be of two or more types.

Examples of the polyolefin backbone include a polyethylene backbone composed only of ethylene, a polypropylene backbone composed of only propylene, and an ethylene-propylene copolymer backbone composed of ethylene and propylene.

When the polyolefin backbone in which one or more hydrogen atoms may be substituted with a fluorine atom is a fluorine-substituted polyolefin, the fluorine-substituted polyolefin is preferably a perfluoropolyolefin in which all hydrogen atoms are substituted with a fluorine atom.

Examples of an alkadiene constituting the polyalkadiene backbone include butadiene, cyclohexadiene, methylcyclohexadiene, cyclooctadiene, methylcyclooctadiene, and phenylcyclooctadiene. The alkadiene constituting the polyalkadiene backbone may be of one type, or may be of two or more types.

The polyphenylene backbone is formed from a structure in which two or more benzene rings are bonded to each other through a single bond.

The specific resin is preferably a resin having a sulfone group and a polyolefin backbone in which one or more hydrogen atoms may be substituted with a fluorine atom [Resin (I)], a resin having a sulfone group and a polyphenylene backbone [Resin (II)], a resin having a sulfone group and a polystyrene backbone [Resin (III)], a resin having a sulfone group and a polyalkadiene backbone [Resin (IV)], a resin having a group represented by Formula (A) above and a polyolefin backbone [Resin (V)], or a resin having a group represented by Formula (A) above and a monovalent hydrocarbon group having two or more carbons in which one or more hydrogen atoms may be substituted with a fluorine atom [Resin (VI)]. The above resin is preferably soluble or dispersible in room temperature water at a concentration of 0.5 mass% or greater.

Examples of Resin (I) include a compound having a polyolefin backbone in which one or more hydrogen atoms may be substituted with a fluorine atom as a main chain and an alkoxy group with a sulfone group bonded to the alkoxy end (that is, a sulfone group bonded to a primary carbon) as a side chain, such as a compound represented by Formula (I-1) below and a compound represented by Formula (I-2) below. Note that one or more hydrogen atoms in the alkoxy group may be substituted with a fluorine atom. Among these, Resin (I) is preferably a resin having polyethylene as a main chain and a 2-sulfoethyloxy group as a side chain, or a resin having perfluoropolyethylene as a main chain and a (2-sulfohexafluoroethyl)oxy group as a side chain.

[Chem. 2]

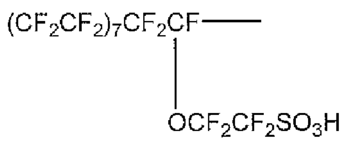
(I-1)

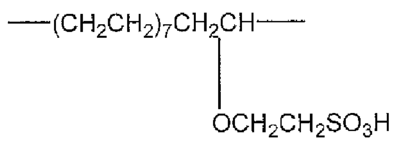
(I-2)

Examples of Resin (II) include a compound having a polyphenylene backbone as a main chain and an aromatic sulfonic acid group in a side chain, such as a compound represented by Formula (II-1) below and a compound represented

by Formula (II-2) below.

[Chem. 3]

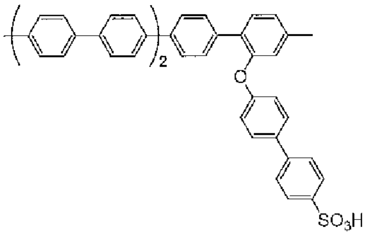

(II-1)

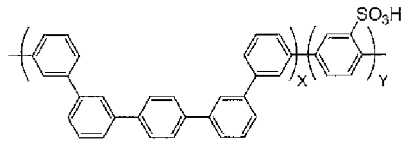

(II-2)

X=1, Y=2

Examples of Resin (III) include a copolymer of styrene and a p-styrenesulfonic acid. The copolymer above may be either a block copolymer or a random copolymer. A molar ratio of a constituent unit derived from styrene to a constituent unit derived from the p-styrenesulfonic acid (the former/the latter) in the copolymer, although not limited, is preferably from 1/100 to 100/1 (that is, from 0.01 to 100.0), more preferably from 0.01 to 90, even more preferably from 0.02 to 80, further preferably from 0.1 to 20, and particularly preferably from 0.5 to 5.

Examples of Resin (III) include a compound represented by Formulas (III-1) to (III-3) below.

[Chem. 4]

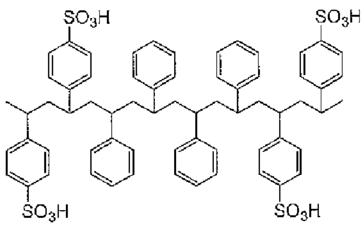

(III-1)

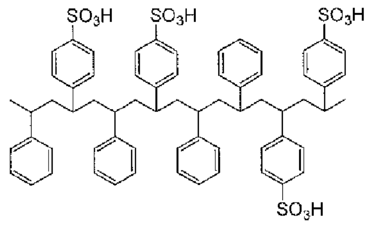

(III-2)

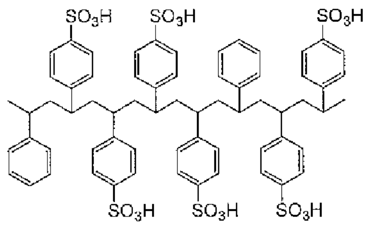

(III-3)

Examples of Resin (IV) include a copolymer of an alkadiene (in particular, butadiene) and a styrenesulfonic acid, or a copolymer of an alkadiene (in particular, butadiene) and vinylsulfonic acid. The butadiene in this case may be either cis-type or trans-type. The copolymer above may be either a block copolymer or a random copolymer. A molar ratio of a constituent unit derived from an alkadiene to a constituent unit derived from a styrenesulfonic acid and/or vinylsulfonic acid (the former/the latter) in the copolymer, although not limited, is preferably from 1/100 to 100/1 (that is, from 0.01 to 100.0), more preferably from 0.01 to 90, even more preferably from 0.02 to 80, further preferably from 0.1 to 20, and particularly preferably from 0.5 to 5.

Examples of Resin (IV) include a compound represented by Formula (IV-1) below and a compound represented by Formula (IV-2) below.

[Chem. 5]

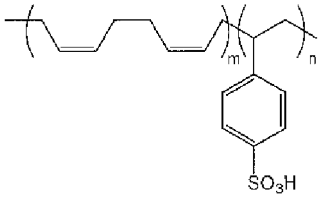

(IV-1)

m=3, n=2

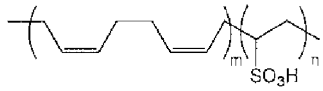

(IV-2)

m=3, n=2

Examples of Resin (V) include a resin having a polyolefin backbone as a main chain and a group represented by Formula (A) above at the end of a side chain. The resin is preferably a resin having a polyethylene backbone as a main chain and a phosphorylcholine group at the end of a side chain. Examples of Resin (V) include a compound represented by Formula (V-1) below.

[Chem. 6]

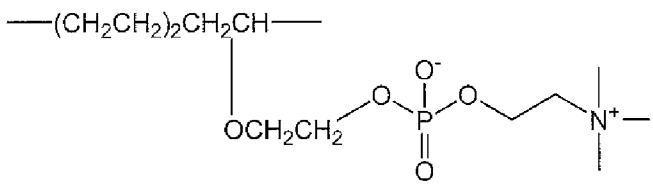

(V-1)

Examples of Resin (VI) include a copolymer of a radically polymerizable monomer having a group represented by Formula (A) above and a (meth)acrylate having, in the ester moiety, a monovalent hydrocarbon group having two or more carbons in which one or more hydrogen atoms may be substituted with a fluorine atom.

Resin (VI) is particularly preferably a copolymer including a constituent unit represented by Formula (1) below and a constituent unit represented by Formula (2) below.

[Chem. 7]

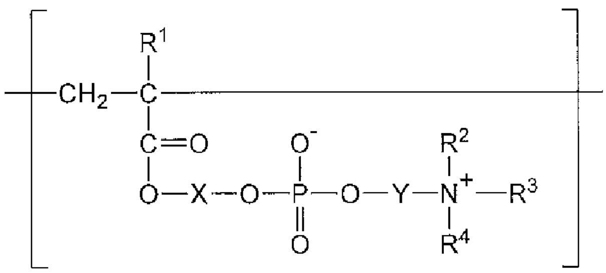

(1)

where in Formula (1), $R^1$ represents a hydrogen atom or an alkyl group having from 1 to 4 carbons; $R^2$, $R^3$, and $R^4$ are the same or different and each represent an alkyl group having from 1 to 4 carbons; X represents a divalent hydrocarbon group having from 1 to 4 carbons; Y represents a divalent linear hydrocarbon group having from 1 to 4 carbons.

[Chem. 8]

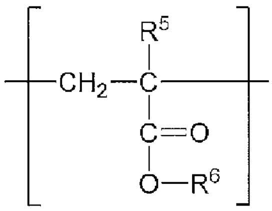

(2)

where in Formula (2), $R^5$ represents a hydrogen atom or an alkyl group having from 1 to 4 carbons, and $R^6$ represents a hydrocarbon group having 2 or more carbons.

By including the constituent unit represented by Formula (1) above, the copolymer has an amphoteric and extremely hydrophilic group. Furthermore, by containing the constituent unit represented by Formula (2) above, the copolymer has an alkyl ester moiety having 2 or more carbons, which is a hydrophobic portion. As such, it is inferred that a structure in which the hydrophilic portion and the hydrophobic portion are phase-separated is more easily formed in the moisture-permeable membrane formed of the copolymer, and that the hydrophilic portion can function as a water-guiding path to allow more water vapor to pass through, resulting in excellent moisture permeability. In addition, regarding the hydrophilic portion and the hydrophobic portion, when the proportion of the hydrophobic portion in the molar ratio is increased, it is also possible to obtain a moisture-permeable membrane that has excellent moisture permeability, is difficult to dissolve in water, and has excellent water resistance. Furthermore, since the pH of the aqueous solution of

the copolymer described above is weakly acidic, the resulting moisture-permeable membrane has an excellent metal corrosion resistance compared to that of a moisture-permeable membrane using a known resin that is strongly acidic and that has an acidic functional group such as a sulfonyl group.

In Formula (1), $R^1$ represents a hydrogen atom or an alkyl group having from 1 to 4 carbons, preferably a hydrogen atom or a methyl group, more preferably a methyl group.

In Formula (1), $R^2$, $R^3$, and $R^4$ are the same or different and each represent an alkyl group having from 1 to 4 carbons. Examples of the alkyl group having from 1 to 4 carbons include a methyl group, an ethyl group, a propyl group, a butyl group, and a t-butyl group. Among these, a methyl group is preferred.

In Formula (1), X represents a divalent hydrocarbon group having from 1 to 4 carbons, and examples thereof include an alkylene group, an alkenylene group, and an alkynylene group. Examples of the alkylene group include a linear or branched $C_{1-4}$ alkylene group, such as a methylene group, a dimethylene group, a trimethylene group, an isopropylene group, and a tetramethylene group. Examples of the alkenylene group include a linear or branched $C_{2-4}$ alkenylene group, such as an ethynylene group, a 1-propenylene group, an isopropenylene group, a 1-butenylene group, a 2-butenylene group, and a 3-butenylene group. Among these, the divalent hydrocarbon group is preferably a linear or branched alkylene group, more preferably a linear alkylene group.

In Formula (1), Y represents a divalent linear hydrocarbon group having from 1 to 4 carbons, and examples thereof include an alkylene group, an alkenylene group, and an alkynylene group. Examples of the alkylene group include a methylene group, a dimethylene group, a trimethylene group, and a tetramethylene group. Examples of the alkenylene group include an ethynylene group, a 1-propenylene group, and a 1-butenylene group. Among these, the divalent linear hydrocarbon group is preferably an alkylene group, and more preferably a dimethylene group.

Examples of a monomer forming the constituent unit represented by Formula (1) above include 2-methacryloyloxyethyl phosphorylcholine.

In Formula (2), $R^5$ represents a hydrogen atom or an alkyl group having from 1 to 4 carbons, preferably a hydrogen atom or a methyl group, and more preferably a methyl group.

In Formula (2), $R^6$ represents a hydrocarbon group having 2 or more carbons. From the viewpoint of a more appropriate hydrophobicity of the hydrophobic portion, the number of carbons is preferably from 4 to 26, more preferably from 8 to 22, even more preferably from 10 to 20, and particularly preferably from 14 to 18.

Examples of the hydrocarbon group having 2 or more carbons include an aliphatic hydrocarbon group, an alicyclic hydrocarbon group, an aromatic hydrocarbon group, and a group in which two or more of those listed above are bonded.

Examples of the aliphatic hydrocarbon group include an alkyl group, an alkenyl group, and an alkynyl group. Examples of the alkyl group include a linear or branched alkyl group, such as an ethyl group, a propyl group, an isopropyl group, a butyl group, a hexyl group, an octyl group, an isooctyl group, a decyl group, a dodecyl group, and a stearyl group. Examples of the alkenyl group include a linear or branched alkenyl group, such as a vinyl group, an allyl group, a methallyl group, a 1-propenyl group, an isopropenyl group, a 1-butenyl group, a 2-butenyl group, a 3-butenyl group, a 1-pentenyl group, a 2-pentenyl group, a 3-pentenyl group, a 4-pentenyl group, and a 5-hexenyl group. Examples of the alkynyl group include a linear or branched alkynyl group, such as an ethynyl group and a propynyl group.

Examples of the alicyclic hydrocarbon group include: a $C_{3-12}$ cycloalkyl group, such as a cyclopropyl group, a cyclobutyl group, a cyclopentyl group, a cyclohexyl group, and a cyclododecyl group; a $C_{3-12}$ cycloalkenyl group, such as a cyclohexenyl group; and a $C_{4-15}$ crosslinked cyclic hydrocarbon group, such as a bicycloheptanyl group and a bicycloheptenyl group.

Examples of the aromatic hydrocarbon group include a $C_{6-14}$ aryl group, such as a phenyl group and a naphthyl group (especially, a $C_{6-10}$ aryl group).

Among these, the hydrocarbon group having 2 or more carbons is preferably an aliphatic hydrocarbon group, more preferably a linear or branched alkyl group, and further preferably a linear alkyl group.

Examples of the monomer forming the constituent unit represented by Formula (2) above include stearyl (meth)acrylate.

Only one type of each of the constituent unit represented by Formula (1) above and the constituent unit represented by Formula (2) above may be contained, or two or more types of each of the constituent unit represented by Formula (1) above and the constituent unit represented by Formula (2) above may be contained.

A molar ratio of the constituent unit represented by Formula (1) above to the constituent unit represented by Formula (2) above (the former/the latter), although not limited, is preferably from 1/100 to 100/1 (that is, from 0.01 to 100.0), more preferably from 0.01 to 90, even more preferably from 0.02 to 80, further preferably from 0.1 to 20, and particularly preferably from 0.5 to 5. It is inferred that when the molar ratio is within the above range, the hydrophilic portion and the hydrophobic portion are present in the copolymer in a well-balanced manner, a structure in which the hydrophilic portion and the hydrophobic portion are phase-separated is more easily formed in the moisture-permeable membrane formed of the copolymer, and the hydrophilic portion can function as a water-guiding path to allow more water vapor to pass through, resulting in superior moisture permeability.

In the copolymer described above, the polymerized forms of the constituent unit represented by Formula (1) above and the constituent unit represented by Formula (2) above are not limited, and may be any of block copolymerization, alternating copolymerization, and random copolymerization. When the copolymer is a copolymer of the monomer forming the constituent unit represented by Formula (1) above and the monomer forming the constituent unit represented by Formula (2) above, the copolymer may be any of a block copolymer, an alternating copolymer, or a random copolymer. Among these, the copolymer is preferably a random copolymer.

The copolymer may have a constituent unit derived from another monomer in addition to the constituent unit represented by Formula (1) above and the constituent unit represented by Formula (2) above. However, in the copolymer, the total number of moles of the constituent unit represented by Formula (1) above and the constituent unit represented by Formula (2) above is preferably 50 mol% or greater, more preferably 90 mol% or greater, and even more preferably 99 mol% or greater, relative to the total number of moles of constituent units derived from all the monomers constituting the copolymer.

Specific examples of Resin (VI) include a copolymer that is a compound represented by Formula (VI-1) below.

[Chem. 9]

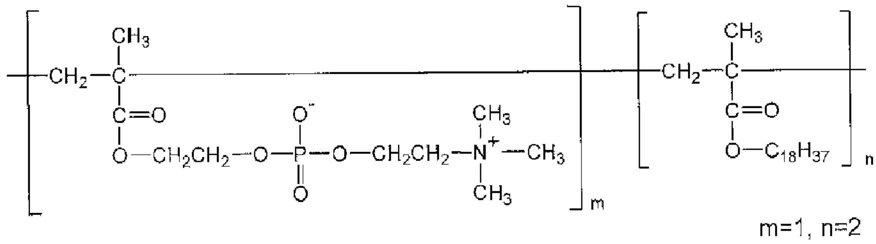

(VI-1)

m=1, n=2

A weight average molecular weight of the copolymer, although not limited, is preferably from 20000 to 2000000, more preferably from 30000 to 1500000, even more preferably from 50000 to 1000000, and particularly preferably from 70000 to 500000. The weight average molecular weight is a value measured by gel permeation chromatography (GPC) and calibrated with polystyrene standards.

The moisture-permeable membrane is formed of the specific resin. The moisture-permeable membrane may include another component in addition to the specific resin within a range that does not impair the effects of the laminate.

A thickness of the moisture-permeable membrane, although not limited, is preferably from 50 to 1000 nm, and more preferably from 100 to 500 nm. When the thickness is 50 nm or greater, the membrane-forming property becomes good, leading to improved gas barrier property. When the thickness is 1000 nm or less, the moisture permeability becomes better. Further, it is easy to form a thin membrane of the moisture-permeable membrane of 1000 nm or less, and cost efficiency is excellent.

As an element that serves as a support of the moisture-permeable membrane, the porous substrate preferably has excellent moisture permeability.

A material that forms the porous substrate may be either a hydrophilic material or a hydrophobic material, but is preferably a hydrophobic material. In a case in which the hydrophobic material is used, when an aqueous composition for forming moisture-permeable membrane is being applied, the aqueous composition does not penetrate into the porous substrate, and as such, it becomes unnecessary to have a lead substrate for preventing the aqueous composition from flowing down from the side opposite to the coating film-formed side of the porous substrate.

Examples of the material that forms the porous substrate include a polyolefin-based resin, a cellulose-based resin, a polycarbonate resin, a polyamide resin, a polyimide resin, a polyamide-imide resin, a fluorine-based resin, an inorganic substance such as metal and glass, and paper. Among these, a polyolefin-based resin is preferable since the moisture-permeable membrane can be formed on the porous substrate at a relatively low temperature and from the viewpoint of excellent moisture permeability and water resistance. The material may be in the form of fibers such as metal fibers and inorganic fibers. The material that forms the porous substrate may be only one type or two or more types.

Examples of the porous material include a resin porous membrane, an inorganic porous membrane, a metal porous membrane, and a fibrous substrate.

The polyolefin-based resin is a polymer (including an olefin-based elastomer) composed of an olefin as an essential monomer component, that is, a polymer containing at least a constituent unit derived from an olefin in a molecule (in one molecule). The olefin is not limited, and examples thereof include an $\alpha$-olefin such as ethylene, propylene, 1-butene, and 4-methyl-1-pentene.

Examples of the polyolefin-based resin include a polymer composed of ethylene as an essential monomer component (polyethylene-based resin), a polymer composed of propylene as an essential monomer component (polypropylene-based resin), an ionomer, and an amorphous cyclic olefin-based polymer. Among these, a polypropylene-based resin is preferable.

A porosity of the porous substrate is not limited, but is preferably from 30 to 80 vol%, and more preferably from 40 to 70 vol%. When the porosity is 30 vol% or greater, moisture permeability improves. When the porosity is 80 vol% or less, the support performance of the moisture-permeable membrane improves.

A thickness of the porous substrate is not limited, but is preferably 5 μm or greater, and more preferably 10 μm or greater, from the viewpoint of allowing the moisture-permeable membrane to be sufficiently supported. In addition, the thickness of the porous substrate is preferably 50 μm or less, and more preferably 30 μm or less, taking into account the viewpoint of excellent moisture permeability and/or cost efficiency.

The surface of a side of the porous substrate (for example, the side 11a illustrated in FIG. 1) on which the moisture-permeable membrane is provided preferably has been subjected to a hydrophilization treatment, from the viewpoint of making it possible to easily form the moisture-permeable membrane. In particular, when a hydrophobic material is used as the material that forms the porous substrate, it is preferable that the hydrophilization treatment is performed. Examples of the hydrophilization treatment include corona discharge treatment and plasma treatment. These hydrophilization treatments allow a carboxy group, a hydroxy group, or a carbonyl group to be generated at a porous substrate surface; as such, the aqueous composition for forming moisture-permeable membrane easily wets the porous substrate surface and spreads on the porous substrate surface, making it easy to form the moisture-permeable membrane. Also, this improves adhesion between the porous substrate and the moisture-permeable membrane. In addition, when the porous substrate formed of a hydrophobic substrate is stored as a roll, the roll is in a form in which one side and the other side of the porous substrate are in contact with each other; since the one side having hydrophilicity and the other side having hydrophobicity are in contact with each other, blocking can be prevented.

A surface tension of the surface of a side of the porous substrate provided with the moisture-permeable membrane is preferably from 35 to 55 dyn/cm, and more preferably from 37 to 50 dyn/cm. When the surface tension is 35 dyn/cm or greater, it becomes easy to apply the aqueous composition for forming moisture-permeable membrane, and the formation of the moisture-permeable membrane becomes easy. When the surface tension is 55 dyn/cm or less, the aqueous composition for forming moisture-permeable membrane does not wet and spread too much, and the moisture-permeable membrane can be easily formed on a porous substrate surface. Note that in a case in which a surface of the porous substrate has been subjected to a hydrophilization treatment, the surface of the side provided with the moisture-permeable membrane is the surface that has undergone the hydrophilization treatment.

A surface tension of the inner part of the porous substrate (that is, a region of the porous substrate in which the moisture-permeable membrane is not formed) is preferably less than 35 dyn/cm, and more preferably 33 dyn/cm or less. When the surface tension is less than 35 dyn/cm, the aqueous composition for forming moisture-permeable membrane is suppressed from penetrating into the inner part of the porous substrate, and the moisture-permeable membrane can be easily formed on a porous substrate surface. Note that in a case in which a surface of the porous substrate has been subjected to a hydrophilization treatment, the inner part of the porous substrate is a region that has not undergone a hydrophilization treatment. Furthermore, the surface tension of the inner part can be obtained by measuring a cross-section obtained by cutting the porous substrate.

The laminate preferably has a moisture permeability of 1600 g/($m^2$·24h) or greater, more preferably 1700 g/($m^2$·24h) or greater, and even more preferably 1800 g/($m^2$·24h) or greater based on the moisture permeability testing method (the cup method) according to JIS Z0208-1976 under the conditions of a temperature of 20°C, a relative humidity of 65%, and a wind speed of 0.2 m/s or less. The laminate has excellent moisture permeability, and thus it is possible to have a configuration in which the moisture permeability is 1600 g/($m^2$·24h) or greater.

The laminate preferably has a moisture permeability of 300 g/($m^2$·24h) or greater, more preferably 400 g/($m^2$·24h) or greater, and even more preferably 500 g/($m^2$·24h) or greater based on the moisture permeability testing method (the cup method) according to JIS Z0208-1976 under the conditions of a temperature of 5°C, a relative humidity of 45%, and a wind speed of 0.2 m/s or less. The laminate has an excellent moisture permeability in a low temperature and low humidity environment, and thus it is possible to have a configuration in which the moisture permeability is 300 g/($m^2$·24h) or greater.

The laminate preferably has an air resistance of 3000 seconds/100 cc or greater, more preferably 4000 seconds/100 cc or greater, and even more preferably 5000 seconds/100 cc or greater based on the Gurley method according to JIS P8117-2009. The laminate has a low air permeability, and thus it is possible to have a configuration in which the air resistance is 3000 seconds/100 cc or greater.

The laminate preferably has a rate of decrease in air resistance according to the following water resistance test of 50% or less, more preferably 20% or less, and even more preferably 15% or less. When the rate of decrease in air resistance is 50% or less, water resistance is superior. In addition, in a case in which the rate of decrease in air resistance is within the range described above while the proportion of the hydrophobic portion in the specific resin is high, water resistance is even better.

Water resistance test

A test piece of φ7 cm is cut out from the laminate, and the air resistance is measured (initial air resistance). Thereafter, the test piece is immersed in 1 L of room temperature water for 15 minutes, and then naturally dried at room temperature. The immersion and drying counts as one cycle, and the cycle is repeated 50 times on the test piece, resulting in a test piece after water resistance test. Then, the air resistance of the obtained test piece after water resistance test (air resistance after water resistance test) is measured. Then, the rate of decrease in air resistance is determined in accordance with the following formula. Note that both the initial air resistance and the air resistance after water resistance test are an air resistance based on the Gurley method according to JIS P8117-2009.

Rate of decrease in air resistance (%) = [(initial air resistance) - (air resistance after water resistance test)]/(initial air resistance) × 100

The laminate preferably has a moisture permeability after the water resistance test of 1600 g/($m^2$·24h) or greater, more preferably 1700 g/($m^2$·24h) or greater, and even more preferably 1800 g/($m^2$·24h) or greater, based on the moisture permeability testing method (the cup method) according to JIS Z0208-1976. The laminate has excellent water resistance and moisture permeability, and thus it is possible to have a configuration in which the moisture permeability after the water resistance test is 1600 g/($m^2$·24h) or greater.

The laminate can be produced by forming the moisture-permeable film on the surface of at least one side of the porous substrate by a known or commonly-used method. For example, the moisture-permeable membrane may be formed directly on the surface of one side of the porous substrate, or the moisture-permeable membrane may be temporarily formed on another support, and then transferred to (bonded with) the surface of one side of the porous substrate to form the moisture-permeable membrane on the porous substrate. Of which, the former method is preferable from the viewpoint of excellent adhesion between the moisture-permeable membrane and the porous substrate.

The surface of a side of the porous substrate on which the moisture-permeable membrane is provided may be subjected to a hydrophilic treatment. Examples of the hydrophilic treatment include those described above.

The moisture-permeable membrane can be formed by applying (coating) a composition for forming the moisture-permeable membrane on the porous substrate or on the other support, and removing solvent from the obtained coating film by heating or the like.

The composition can be produced by a known or commonly used method. For example, the composition can be produced by dissolving or dispersing the specific resin in a solvent and, as needed, mixing in an additive. The solvent is preferably water and/or a water-soluble solvent. When water or a water-soluble solvent is used, it is inferred that the specific resin is dispersed in the composition in a core-shell shape in which the inner side is a hydrophobic portion while the outer side is a hydrophilic portion. By using such a composition, when the coating film is dried, the hydrophilic part and the hydrophobic part are phase-separated to form a moisture-permeable film having a water guide path, and it is inferred that the hydrophobic parts are firmly bonded to each other, leading to better water resistance.

Examples of the water-soluble solvent include: an aliphatic water-soluble alcohol, such as methanol, ethanol, n-propanol, and i-propanol; and a glycol ether, such as ethylene glycol monomethyl ether, ethylene glycol monobutyl ether, propylene glycol monomethyl ether, and propylene glycol monoethyl ether. One of the water-soluble solvents listed above may be used, or two or more thereof may be used.

A proportion (concentration) of the specific resin in the composition in which the specific resin is dissolved or dispersed, although not limited, is preferably from 0.5 to 5 mass%, more preferably from 1 to 4 mass%, and even more preferably from 1.5 to 3 mass%. When the concentration is 5 mass% or less, the thickness of the coating layer increases, and thus the film thickness of the moisture-permeable membrane after drying is more uniform. This makes it possible to form a moisture-permeable membrane that is thinner while having an excellent gas barrier property, and consequently, the moisture permeability is further improved. Furthermore, when the concentration is within the above range, it is easy to form a moisture-permeable membrane having excellent coatability as well as excellent moisture permeability and gas barrier property.

Note that a known coating method may be used for the application (coating) of the composition. For example, a coater such as a gravure roll coater, a reverse roll coater, a kiss roll coater, a dip roll coater, a bar coater, a knife coater, a spray coater, a comma coater, or a direct coater may be used.

The heating temperature when removing solvent from the coating film is preferably from 35 to 90°C, more preferably from 40 to 85°C, and even more preferably from 45 to 80°C. The heating time may be a suitable time employed as appropriate, but is, for example, from 5 seconds to 20 minutes, preferably from 5 seconds to 10 minutes, and more preferably from 10 seconds to 5 minutes. Since a moisture-permeable membrane can be formed at a temperature as low as 90°C or less (in particular, 80°C or less) using the composition, film formation is easy, and a polyolefin-based resin having excellent moisture permeability can be used as the porous substrate.

The laminate has a low air permeability and an excellent moisture permeability. Furthermore, the laminate has an excellent moisture permeability and water resistance in a low temperature and low humidity environment. As such, the laminate can be preferably used in a product that requires such a function, such as a total heat exchange device, an undergarment, a disposable water-repellent/moisture-permeable material, and an application for dehydration without exposure to air or bacteria (a filter for storing aged meat, etc.), a product for improving the environment (air and water quality), and a separation adsorption material. The laminate for total heat exchanger device is, for example, a sheet capable of exchanging temperature (sensible heat) and moisture (latent heat) between supply air and exhaust air. Further, when the laminate has a structure in which the hydrophilic portion and the hydrophobic portion are phase-separated, the laminate can also be used as a barrier film having moisture permeability. When the diameter of the hydrophilic portion is small, the barrier film having moisture permeability allows a hydrophilic substance that is small in size (for example, water vapor) to pass through the hydrophilic portion while not allowing a substance that is large in size (for example, a virus) to pass through, making it possible to separate the two.

By changing the shape of the laminate into a corrugated shape as needed and further performing lamination, the laminate can be turned into a total heat exchange device (total heat exchange sheet). The total heat exchanger device may be either a cross-flow type or a counter-flow type. A total heat exchange device using the laminate described above has a low air permeability, an excellent moisture permeability (in particular, moisture permeability in a low temperature and low humidity environment), and an excellent water resistance. The total heat exchanger device can be used as a total heat exchange device of an air conditioner. The air conditioner uses a total heat exchange device having a low air permeability, an excellent moisture permeability, as well as an excellent moisture permeability in a low temperature and low humidity environment, and an excellent water resistance; as such, the air conditioner has an excellent indoor heat retention, an excellent indoor moisture retention, and an excellent durability even in a low temperature and low humidity environment.

Calculation method of and calculation device for attraction value and repulsive value relative to water

The present disclosure also provides a method of calculating an attraction value and a repulsive value relative to a solvent of a target molecule. The calculation method includes: a solvation system creation step of arranging the target molecule in a cubic imaginary space and arranging the solvent molecules around the target molecule in the imaginary space; a molecular dynamics calculation step of establishing an equilibrium state of the solvation system at room temperature and normal pressure; a histogram creation step of creating, in the imaginary space, an energy histogram of a combination of solute and solvent in the equilibrium state in a process of calculating free energy based on energy representation; and an integration step of calculating a negative integral value in the obtained energy histogram as the attraction value relative to solvent of the target molecule and calculating a positive integral value in the obtained energy histogram as the attraction value relative to solvent of the target molecule.

When water is used as the solvent, an attraction value and a repulsive value relative to water can be calculated using the calculation method described above. In this way, it is possible to determine whether or not the target molecule corresponds to the specific resin described above.

Furthermore, the target molecule is preferably a molecule having a hydrophilic portion and a hydrophobic portion. When the solvent is water while the target molecule is a molecule having a hydrophilic portion and a hydrophobic portion, an attraction value and a repulsive value relative to water can be more suitably calculated by the calculation method described above.

FIG. 2 illustrates a flow chart according to the calculation method of an embodiment. The embodiment described above includes, in this order, a target molecule structure modeling step S1, a stable conformation search step S2, a structural optimization calculation step S3, a force field parameter generation step S4, an energy minimization calculation step S5, a solvation system creation step S6, a molecular dynamics calculation step S7, a histogram creation step S8, and an integration step S9.

In the target molecule structure modeling step S1, the three-dimensional molecular structure of a target molecule is modeled. Next, in the stable conformation search step S2, the modeled target molecule is subjected to a conformation search based on molecular mechanics to determine a stable conformation at the classical-mechanics level. Note that, when the stable conformation of the target molecule can be determined based on chemical experience, the stable conformation search step S2 may be omitted.

The target molecule structure modeling step S1 and the stable conformation search step S2 can be performed using a computational chemistry integration platform SCIGRESS 2.9. Stable conformation search can be performed, for example, using the CONFLEX function and employing MM3 for the molecular force field.

In the structural optimization calculation step S3, the optimized structure in vacuum is calculated for the target molecule that has undergone the stable conformation search step S2 as necessary. Specifically, the Cartesian coordinates corresponding to the stable conformation at the classical-mechanics level are prepared as the input data, and structural optimization calculation is performed based on the density functional theory. The structural optimization calculation based on the density functional method described above can be performed, for example, using the quantum chemistry calculation software Gaussian 16. The calculation method/basis function is preferably set to B3LYP/6-31G(d), and the SCF calculation convergence condition is preferably set to "SCF = tight". Note that the optimized structure obtained in the step S3 is used to calculate the partial charge on each atom in the force field parameter generation step S4.

In the force field parameter generation step S4, the partial charge (Coulomb force) on each atom in the optimized structure of the target molecule obtained in the structural optimization calculation step S3 is calculated to generate force field parameters. Force field parameter generation can be performed, for example, using the quantum chemistry calculation software Gaussian 16. Regarding the force field parameters, in addition to the Coulomb force, it is determined that the interatomic bond, bond angle, dihedral angle, and vdW force are general ones, and the general AMBER force field 2 (Gaff2) can be applied. The Coulomb force is calculated in the step S4, as it is the type of interaction that has the greatest impact on the solvation system.

In the energy minimization calculation step S5, energy minimization calculation of the target molecule is performed while taking into consideration the force field parameters generated in the force field parameter generation step S4. A known or commonly used molecular dynamics simulation package, such as NAMD 12.0, AMBER, or Gromacs, may be used to perform the energy minimization calculation.

In the solvation system creation step S6, a solvation system is created by arranging the target molecule in a cubic imaginary space and arranging solvent molecules around the target molecule in the imaginary space. Specifically, for example, a topology file and a coordinate file of the initial solvation system are created in accordance with the following procedure. First, centering is performed to align the center of gravity of the target molecule having the minimum energy structure with the origin. Then, the centered target molecule is arranged in a cubic imaginary space, and solvent molecules are randomly arranged in a region inside the imaginary space that is beyond 2Å from the surface of the centered target molecule so that the density is smaller than the room temperature density. A size of the cube of the imaginary space is preferably set to approximately from 8000 to 12000 (in particular, from 9000 to 10000) water molecules. When the number of water molecules is within the above range, the minimum system size required by the solvation system can be sufficiently ensured even for molecules having a large maximum molecular diameter such as styrene-sulfonic acids. The topology/coordinate file of an initial hydration system and the PDB file can be generated using, for example, the Leap module of AmberTools 16 and the "tleap" command. Note that density is determined automatically by AmberTools 16.

In the molecular dynamics calculation step S7, a molecular dynamics calculation is performed to establish an equilibrium state of the solvation system at room temperature and normal pressure. The molecular dynamics calculation step S7 preferably includes a first to fifth calculation phases below.

(i) a first calculation phase, in which a molecular dynamics calculation is performed at a temperature of over 300 K, a pressure of 1 MPa or greater, and with an addition of an energy constraint of 1 kcal/(mol·Å$^2$) or greater to a minimum energy structure
(ii) a second calculation phase after the first calculation phase, in which a molecular dynamics calculation is performed while the addition of the energy constraint is reduced until the addition reaches 0 kcal/(mol·Å$^2$)
(iii) a third calculation phase after the second calculation phase, in which a molecular dynamics calculation is performed while the pressure is reduced until the pressure reaches 1 atm
(iv) a fourth calculation phase after the third calculation phase, in which a molecular dynamics calculation is performed while the temperature is reduced until the temperature reaches 300 K
(v) a fifth calculation phase after the fourth calculation phase, in which a molecular dynamics calculation is performed at a temperature of 300 K, a pressure of 1 atm, and with the energy constraint lifted, resulting in an equilibrium state of the solvation system at room temperature and normal pressure

A known or commonly used molecular dynamics simulation package, such as NAMD 12.0, AMBER, or Gromacs, may be used to perform all of the first to fifth calculation phases.

The first calculation phase is a phase of performing a molecular dynamics calculation under conditions of high temperature, high pressure, and addition of constraint. In the first calculation phase, to enhance the relaxation with surrounding solvent molecules while maintaining the minimum energy structure of the target molecule, a molecular dynamics calculation is performed at a temperature of over 300 K, a pressure of 1 MPa or greater (such as from 1 to 40 MPa, preferably from 10 to 20 MPa), and with an addition of an energy constraint of 1 kcal/(mol·Å$^2$) or greater [such as from 5 to 20 kcal/(mol·Å$^2$)] to the minimum energy structure. Also, the molecular dynamics calculation is executed, for example, at a time step of 2 fs and a total of 20 ps. Note that the procedure of the first calculation phase may be omitted if the target molecular structure does not have higher-order structure characteristics, and the higher the higher-order structure characteristics, the higher the temperature, up to approximately 600 K, at which the calculation is performed.

The second calculation phase is a phase of performing a molecular dynamics calculation under conditions of

high temperature, high pressure, and without constraint. In the second calculation phase after the first calculation phase, a molecular dynamics calculation is performed while the addition of the energy constraint is reduced until the addition reaches 0 kcal/(mol·Å$^2$). Specifically, the final coordinates and velocity data obtained in the first calculation phase are used as the input data to execute a molecular dynamics calculation for gradually lifting the imaginary constraints. The lifting process is set to include a total number of n calculation processes with values decreasing to 0 kcal/(mol·Å$^2$) by decrements of 1 kcal/(mol·Å$^2$), for example, each process performing an × 10 ps equilibration process. The density of the solvent molecules transitions from a low-density state to a high-density state by going through the first calculation phase, and thus at this time, the length of one side and the number of grids of the solvation system are adjusted based on the size of the basic cell after the first calculation phase. At this time, it is necessary that the number of grids is greater than or equal to the system size. Note that the second calculation phase is omitted when the first calculation phase is not performed.

The third calculation phase is a phase of performing a molecular dynamics calculation during a process of high temperature and reducing pressure. In the third calculation phase after the second calculation phase, a molecular dynamics calculation is performed while the pressure is reduced until the pressure reaches 1 atm. Specifically, the final coordinates, velocity, and basic cell data obtained in the second calculation phase are used as the input data, and a total number of n calculation processes are set to be executed with the pressure reduced from the high pressure condition to the normal pressure 1 atm (0.101325 MPa) by decrements of 1 MPa, for example, each process performing an × 10 ps equilibration process.

The fourth calculation phase is a phase of performing a molecular dynamics calculation in a process of reducing temperature and normal pressure. In the fourth calculation phase after the third calculation phase, a molecular dynamics calculation is performed while the temperature is reduced until the temperature reaches 300 K. Specifically, the final coordinates, velocity, and basic cell data obtained in the third calculation phase are used as the input data, and a total number of n calculation processes are set to be executed with the temperature reduced from the high temperature condition to room temperature 300 K by decrements of 5 K, for example, each process performing an × 10 ps equilibration process.

The fifth calculation phase is a phase of performing a molecular dynamics calculation under conditions of room temperature and normal pressure. In the fifth calculation phase after the fourth calculation phase, a molecular dynamics calculation is performed at a temperature of 300 K, a pressure of 1 atm, and with the energy constraint lifted, resulting in an equilibrium state of the solvation system at room temperature and normal pressure. Specifically, the final coordinates, velocity, and basic cell data obtained in the fourth calculation phase are used as the input data to perform a molecular dynamics calculation under the conditions of room temperature and normal pressure. For example, an equilibration run and a production run for sampling are executed. Note that the total calculation time is appropriately set depending on the state of equilibrium based on the target molecular structure.

In the histogram creation step S8, an energy histogram of a combination of solute and solvent in the equilibrium state by free energy calculation based on energy representation in the imaginary space is created. Specifically, a trajectory file consisting of multiple snapshots of the equilibrated solvation system is generated from the production run obtained in the fifth calculation phase. These trajectories are used to generate an energy histogram of a combination of solute and solvent. Free-energy calculation software ERmod 0.3.5 based on energy representation can be used in the creation.

In the integration step S9, a negative integral value in the obtained energy histogram is calculated as the attraction value relative to solvent of the target molecule, and a positive integral value in the obtained energy histogram is calculated as the attraction value relative to solvent of the target molecule.

In this way, the attraction value and the repulsive value relative to solvent of a target molecule can be calculated.

The present disclosure also provides a calculation device for calculating an attraction value and a repulsive value relative to solvent of a target molecule. The calculation device includes: a solvation system creation portion that arranges the target molecule in a cubic imaginary space and arranges the solvent molecules around the target molecule in the imaginary space; a molecular dynamics calculation portion that establishes an equilibrium state of the solvation system at room temperature and normal pressure; a histogram creation portion that creates, in the imaginary space, an energy histogram of a combination of solute and solvent in the equilibrium state by free energy calculation based on energy representation; and an integration portion that calculates a negative integral value in the obtained energy histogram as the attraction value relative to solvent of the target molecule and calculates a positive integral value in the obtained energy histogram as the attraction value relative to solvent of the target molecule.

An embodiment of the calculation device includes, in this order, a target molecule structure modeling portion, a stable conformation search portion, a structural optimization calculation portion, a force field parameter generation portion, an energy minimization calculation portion, a solvation system creation portion, a molecular dynamics calculation portion, a histogram creation portion, and an integration portion. The target molecule structure modeling portion, the stable conformation search portion, the structural optimization calculation portion, the force field parameter generation portion, the energy minimization calculation portion, the solvation system creation portion, the molecular dynamics calculation portion, the histogram creation portion, and the integration portion each is an element for performing each

of the target molecule structure modeling step S 1, the stable conformation search step S2, the structural optimization calculation step S3, the force field parameter generation step S4, the energy minimization calculation step S5, the solvation system creation step S6, the molecular dynamics calculation step S7, the histogram creation step S8, and the integration step S9 in the calculation method described above. Each of the elements described above in the calculation device is an element suitable for executing a preferred aspect of each of the steps in the calculation method.

Using the calculation method according to an embodiment of the present disclosure and the calculation device according to an embodiment of the present disclosure, an attraction value and a repulsive value relative to solvent of a target molecule can be calculated, and the attraction value can be used as an indicator of philicity while the repulsive value can be used as an indicator of phobicity. Based on this, the solubility in a solvent of a target molecule and the resistance to a solvent of a target molecule, and in turn, the balance therebetween, can be inferred.

Each aspect disclosed in the present specification can be combined with any other feature disclosed herein. Note that each of the configurations, combinations thereof, or the like in each of the embodiments are examples, and additions, omissions, replacements, and other changes to the configurations may be made as appropriate without departing from the spirit of the present disclosure. In addition, each aspect of the invention according to the present disclosure is not limited by the embodiments or the following examples but is limited only by the claims.

Examples

An embodiment of the present invention will be described in further detail below based on examples.

Comparative Example A1

Calculation of attraction value and repulsive value relative to water

An attraction value and a repulsive value relative to water were calculated for a product under the trade name "Nafion" (available from DuPont) as follows.

Target molecule structure modeling step

First, when modeling the three-dimensional molecular structure of Nafion, the computational chemistry integration platform SCIGRESS 2.9 was used to model the three-dimensional structure in Workspace.

Stable conformation search step

After beautifying the three-dimensional structure with a simple beautifying function, a conformation search based on molecular mechanics was carried out using the CONFLEX function with MM3 as the molecular force field to determine a stable conformation at the classical-mechanics level.

Structural optimization calculation step

Next, to determine the optimized structure of Nafion in vacuum, the Cartesian coordinates corresponding to the stable conformation at the classical-mechanics level were prepared as the input data, and structural optimization calculation was performed based on the density functional theory using the quantum chemistry calculation software Gaussian 16. The calculation method/basis function were set to B3LYP/6-31G(d), and the SCF convergence condition was set to "SCF = Tight".

Force field parameter generation step

To calculate the partial charge on each atom in the optimized structure in vacuum, the log file exported after executing the structural optimization calculation by Gaussian 16 was loaded, and single-point calculation based on the density functional theory was performed. At that time, "geom = check guess = read", "pop = mk iop (6/50 = 1, 6/42 = 6)", and "SCF = Tight" were added to the route section to generate an esp file. The generated esp file was used as the input data, and the Antechamber module (antechamber command) that is an accessory module in the AmberTools 16.0 was used to convert the ESP charges into RESP charges. The necessary command references used at this time include: "-fi gesp" for loading the esp file, "-fo prepi" for generating a prep file to be described later, "-c resp" for converting to RESP charges, and "-at gaff2" for indicating the reference force field parameters. The prep file generated as the result contained Z-matrix coordinates converted from the Cartesian coordinates of the optimized structure, RESP charges, arbitrarily specified residue names, bonding information, etc. Then, the prep file was used as the input data, and the

parmchk command that is a part of AmberTools 16.0 was used to search for force field data (atomic mass, parameters of stretching vibration, bending vibration, dihedral vibration, and the L-J parameters) not provided in the gaff2 force field serving as a reference force field, and the results were generated as a frcmod file. Through the above operation, two types of force field parameter files (prep file, frcmod file) and a PDB file of the optimized structure were generated.

Energy minimization calculation step

An Amber topology file and a coordinate file were generated using the two types of parameter files and the PDB file described above as well as the Gaff2 force field as the input data. AmberTools 16 was used to carry out the present operation. Using the molecular dynamics simulation package NAMD 12.0 and the topology file and the coordinate file as input files, an energy minimization calculation under the conditions described later was performed, and the energy minimum structure was converted to a PDB file by the cpptraj command that is a part of AmberTools 16 described above.

The execution conditions for the energy minimization calculation were as follows. In the input file of the NAMD software, "minimization" was set to ON, "minTinyStep" was set to 1.0e-6, "minBabyStep" was set to 1.0e-2, "minLineGoal" was set to 1.0e-4, "reinitvels" was set to 0, and "minimize" was set to 100000. The cutoff distance was set to 12 Å, and the switching function was set to off. Regarding the 1-4 interactions, "exclude" was set to scaled 1-4 and "1-4 scaling" was set to 0.833333.

Solvation system creation step

A topology file and a coordinate file of the initial hydration system were generated in accordance with the following procedure. First, centering was performed to align the center of gravity of the minimum energy structure with the origin. Then, a BOX-type basic cell (imaginary space), that was 20 Å away on the X axis, 28 Å away on the Y axis, and 30 Å away on the Z axis from the surface 2 Å away from the surface of the centered solute, was prepared. Next, water was randomly arranged in the imaginary space to achieve a density of 0.739 g/cc. The TIP3P water model was used as the water model, 9391 water molecules were arranged under these conditions, and a topology file, a coordinate file, and a PDB file of the initial hydration system were generated based on the system. The imaginary space had a size of 71 Å on the X axis, 71 Å on the Y axis, and 71 Å on the Z axis, and a cubic shape. Note that the Leap module of AmberTools 16 and the "tleap" command were used to generate these files. At this time, the two types of force field parameter files described above and the PDB file corresponding to the minimum energy structure were used, and "leaprc.water.tip3p" that is a part of AmberTools 16 was used for the force field of TIP3P.

Molecular dynamics calculation step

A molecular dynamics calculation was performed to generate an equilibrium state at room temperature and normal pressure based on the initial hydration system established. When executing the molecular dynamics calculation, the following procedures of from STEP1 to STEP4 were adopted. NAMD12.0 was used for all runs.

STEP 1: molecular dynamics calculation under conditions of high temperature, high pressure, and addition of constraint

To increase the relaxation with the surrounding water while maintaining the minimum energy structure of Nafion, a molecular dynamics calculation was performed at a time step of 2 fs and a total of 20 ps at a temperature of 320 K, a pressure of 10 MPa, and with an addition of a constraint of 10 kcal/(mol. $Å^2$) to the minimum energy structure. The following information was contained in the input file of the NAMD software. In order for the NAMD software to be able to read the AMBER topology file and coordinate file, "amber" was set to on, and the AMBER topology file and coordinate file were each designated as "parmfile" and "ambercoor". "Firsttimestep" was set to 0, and "cellBasisVector1", "cellBasisVector2", and "cellBasisVector3" were all set to 71. Since the initial hydration system had been centered, "CenterX", "CenterY", and "CenterZ" were all set to 0. "wrapAll" was set to on in order to place the atoms that moved from the basic cell to the periodic boundary image cell during the execution of the molecular dynamics calculation back into the basic cell and generate the coordinate data. The calculation of long-range interactions was set to use the particle mesh Ewald method. "PMEGridSizeX", "PMEGridSizeY", and "PMEGridSizeZ" were all set to 72, the cutoff distance was set to 12 Å, and the witching function was set to off. Regarding the 1-4 interactions, "exclude" was set to scaled 1-4, "1-4scaling" was set to 0.833333, and "margin" was set to 4. In order to perform calculation based on the shake method, "rigidBonds" was set to all, and "rigidTolence" was set to 0.00001. The temperature control was set to perform in accordance with Langevin dynamics, "langevinTemp" was set to 320, and "langevinDamping" was set to 5. The pressure control was set to use the Nose-Hoover Langevin piston method, "useGroupPressure" was set to yes, "use-FlexibleCell" was set to no, "langevinPistonTarget" was set to 10.0, "langevinPistonPeriod" was set to 200.0, "langevin-PistonDecay" was set to 100.0, and "langevinPistonTemp" was set to 320. Note that, in order to add a constraint to the

energy minimum structure of Nafion, "constraints" was set to on, "consexp" was set to 2, and "constraintScaling" was set to 1.

STEP 2: molecular dynamics calculation under conditions of high temperature, high pressure, and without constraint

The final coordinates and velocity data obtained in STEP 1 were used as the input data to perform a molecular dynamics calculation for gradually lifting the imaginary constraints. The lifting process was set to include a total number of 10 calculation processes with values decreasing from 9 to 0 kcal/(mol·$Å^2$) by decrements of 1 kcal/(mol·$Å^2$), each process performing a 10 ps equilibration process. The state transitions from a low-density state of 0.739 g/cc to a high-density state by going through STEP 1, and thus at this time, the size of the basic cell after STEP 1 was 67 Å on the X axis, the Y axis, as well as the Z axis; in addition, the values of "cellBasisVector1", "cellBasisVector2" and "cellBasisVector3" were set to 67, and "PMEGridSizeX", "PMEGridSizeY" and "PMEGridSizeZ" were set to 70. Other settings, temperature control, pressure control method, and calculation conditions were in accordance with STEP 1.

STEP 3: Molecular dynamics calculation during a process of high temperature and reducing pressure

The final coordinates, velocity, and basic cell data obtained in STEP 2 were used as the input data, and a total number of 10 calculation processes were set to be executed with the pressure reduced from a high pressure of 9 MPa to 8, 7, ..., 2, 1, and to the normal pressure 0.101325 MPa by decrements of 1 MPa, each process performing a 10 ps equilibration process. Other settings, temperature control, pressure control method, and calculation conditions were in accordance with STEP 2.

STEP 4: Molecular dynamics calculation in a process of reducing temperature and normal pressure

The final coordinates, velocity, and basic cell data obtained in STEP 3 were used as the input data, and a total number of 4 calculation processes were set to be executed with the temperature reduced from a high temperature of 315 K to room temperature 300 K by decrements of 5 K, each process performing a 10 ps equilibration process. Other settings, temperature control, pressure control method, and calculation conditions were in accordance with STEP 3.

STEP 5: Molecular dynamics calculation under conditions of room temperature and normal pressure

The final coordinates, velocity, and basic cell data obtained in STEP 4 were used as the input data, and a molecular dynamics calculation under the conditions of room temperature and normal pressure was performed for 10 ns for equilibration and 2 ns for a production run for sampling. In the production run, dumpling of the trajectory file was performed at 100 fs, and "DCDfreq" was set to 50. Other settings, temperature control, pressure control method, and calculation conditions were in accordance with STEP 4.

Histogram creation step

From the 2 ns production run obtained in STEP 5, a trajectory file consisting of snapshots of a total of 20000 equilibrated hydration systems was generated. These trajectories were used to generate an energy histogram of a combination of solute and solvent using the analysis procedure described below. Free-energy calculation software ERmod 0.3.5 based on energy representation was used in the creation. First, all trajectories obtained in the production run were centered using the "ptraj" command of AmberTools 16 to align the center of gravity of Nafion with the origin. After that, a group of files (SltInfo, MolPrm1, MDinfo, LJTable) necessary for the calculation of energy histogram were automatically generated by assigning the topology file of the hydration system using the "gen_structure" command of the attached module of ERmod. "20000" was manually entered in the first line and first column of Mdinfo to manually create a parameter file "parameters_er" containing calculation conditions, etc. For energy calculation parameters at that time, "boxshp" was set to 1, "engdiv" was set to 20, and "ljformat" was set to 5. For the energy bin definition parameters, "eclbin" was set to 5.0e-2, "ecfbin" was set to 2.0e-3, "ec0bin" was set to 2.0e-4, "finfac" was set to 10.0e0, "ecdmin" was set to - 50, "ecfmns" was set to -0.20e0, "ecdcen" was set to 0.0e0, "eccore" was set to 20.0e0, "ecdmax" was set to 1.0e11, and "pecore" was set to 200. The "ermod" command of the attached module of ERmod was used to execute the analysis.

Integration step

The attraction value and repulsive value relative to water of Nafion were calculated using the values in the first column of the engsln.20 file (corresponding to the x-axis of the histogram) and the values in the third column (corresponding to the y-axis of the histogram) of the engsln.20 file resulting from the above analysis. The attraction value was defined as the sum of positive values among the products of the first and third columns, and the repulsive value was defined as the sum of negative values. As the calculation result, the attraction value was -106.5 (kcal/mol) while the repulsive value was 34.0 (kcal/mol).

Examples A1 to A11 and Comparative Examples A2 to A11

The attraction values and repulsive values relative to water were calculated in the same manner as in Comparative Example A1 for each of the resins described in Table 1. The results are presented in Table 1.

[Table 1]

Table 1

| | Target Molecule | Attraction Value [kcal/mol] | Repulsive Value [kcal/mol] |
|---|---|---|---|
| Example A1 | Compound represented by Formula (I-1) | -111.9 | 43.8 |
| Example A2 | Compound represented by Formula (I-2) | -115.5 | 56.0 |
| Example A3 | Compound represented by Formula (II-1) | -151.4 | 57.3 |
| Example A4 | Compound represented by Formula (II-2) | -2001 | 90.0 |
| Example A5 | Compound represented by Formula (III-1) | -266.5 | 105.0 |
| Example A6 | Compound represented by Formula (III-2) | -342.1 | 142.8 |
| Example A7 | Compound represented by Formula (III-3) | -446.6 | 183.4 |
| Example A8 | Compound represented by Formula (IV-1) | -205.4 | 95.4 |
| Example A9 | Compound represented by Formula (IV-2) | -152.7 | 51.1 |
| Example A10 | Compound represented by Formula (V-1) | -263.5 | 145.8 |
| Example A11 | Compound represented by Formula (VI-1) | -289.0 | 121.0 |
| Comparative Example A1 | 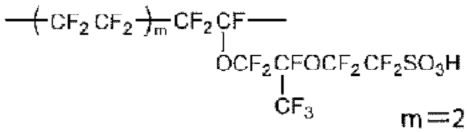 m=2 | -106.5 | 34.0 |
| Comparative Example A2 | 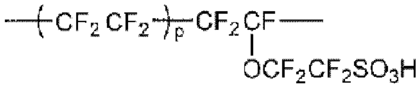 p=2 | -91.7 | 46.5 |
| Comparative Example A3 | 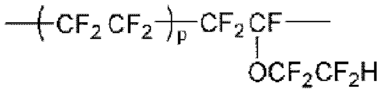 p=2 | -45.5 | 17.8 |
| Comparative Example A4 | 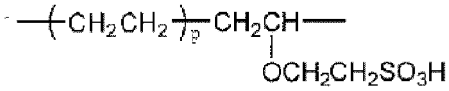 p=2 | -95.6 | 54.1 |
| Comparative Example A5 | 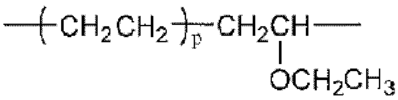 p=2 | -34.1 | 12.8 |

(continued)

| | Target Molecule | Attraction Value [kcal/mol] | Repulsive Value [kcal/mol] |
|---|---|---|---|
| Comparative Example A6 | 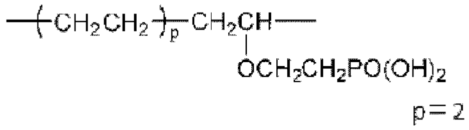 | -91.7 | 33.2 |
| Comparative Example A7 | Cellulose | -94.1 | 38.2 |
| Comparative Example A8 | 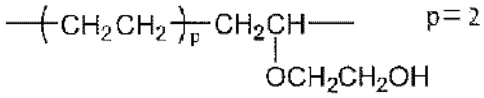 | -48.5 | 19.4 |
| Comparative Example A9 | 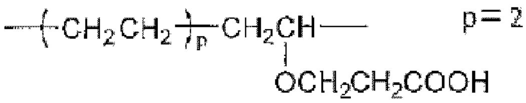 | -55.9 | 19.8 |
| Comparative Example A10 | 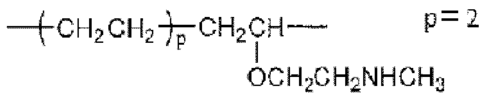 | -50.2 | 18.6 |
| Comparative Example A11 | 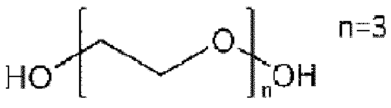 | -86.1 | 39.7 |

The resins of Examples A1 to A11 had an attraction value relative to water of -110 kcal/mol or less and a repulsive value relative to water of 35 kcal/mol or greater, indicating that the resins of Examples A1 to A11 can be preferably used as a resin for making the moisture-permeable membrane in the laminate according to an embodiment of the present disclosure.

Example B1

A random copolymer of 2-methacryloyloxyethyl phosphorylcholine and stearyl methacrylate [ratio of constituent units (former/latter): 1/1, concentration: 4 mass%, weight average molecular weight: 100000) and a preservative were mixed and diluted with distilled water, resulting in a composition having a copolymer concentration of 2 mass% (with the copolymer being the main component in the solid content). Meanwhile, the surface of one side of a porous substrate made of a polyolefin-based resin (thickness: 20 μm, porosity: 48 vol%, surface tension: 32 dyn) was subjected to a corona treatment, resulting in a hydrophilic surface having a surface tension of 46 dyn. Then, the hydrophilic surface of the porous substrate was coated with the composition described above using an applicator, and heating was performed at 50°C for 3 minutes, resulting in a moisture-permeable membrane (thickness: from 100 to 500 nm). In this way, the laminate of Example B1 was produced.

Example B2

The laminate of Example B2 was produced in the same manner as in Example B1 except that a porous substrate made of a polyolefin-based resin (thickness: 12 μm, porosity: 40 vol%, surface tension: 32 dyn) was used as the porous substrate.

Example B3

The laminate of Example B3 was produced in the same manner as in Example B1 except that a porous substrate made of a polyolefin-based resin (thickness: 25 μm, porosity: 56 vol%, surface tension: 32 dyn) was used as the porous substrate.

Example B4

The laminate of Example B4 was produced in the same manner as in Example B1 except that a porous substrate made of a polyolefin-based resin (thickness: 5 μm, porosity: 65 vol%, surface tension: 32 dyn) was used as the porous substrate.

Example B5

The laminate of Example B5 was produced in the same manner as in Example B1 except that the moisture-permeable membrane was formed using a composition of 1.5 mass% obtained by diluting a random copolymer of 2-methacryloyloxyethyl phosphorylcholine and stearyl methacrylate [ratio of constituent units (former/latter): 1/1, concentration: 4 mass%, weight average molecular weight: 100000) with distilled water.

Comparative Example B 1

The laminate of Comparative Example B1 was produced in the same manner as in Example B1 except that the resin of Comparative Example A7 was used instead of the copolymer described above.

Comparative Example B2

The laminate of Comparative Example B2 was produced in the same manner as in Example B1 except that the resin of Comparative Example A1 was used instead of the copolymer described above.

Comparative Example B3

The laminate of Comparative Example B3 was produced in the same manner as in Example B1 except that the resin of Comparative Example A11 was used instead of the copolymer described above.

Evaluation

Each of the laminates obtained in Examples and Comparative Examples was evaluated as follows. The evaluation results are listed in a table. Note that the symbol "-" in the table indicates that evaluation was not performed. Furthermore, a porous substrate (thickness: 20 μm, porosity: 48 vol%, surface tension: 32 dyn) made of a polyolefin-based resin on which a moisture-permeable membrane was not formed was evaluated as Comparative Example B4.

(1) Air resistance

The air resistance of the laminates obtained in Examples B 1 to B5 and Comparative Examples B1 to B4 was measured based on the Gurley method according to JIS P8117-2009. Specifically, a 5 cm × 5 cm test piece was cut out from the laminates obtained in Examples and Comparative Examples and placed in a Gurley device, and the number of seconds needed to pass 100 cc of air was measured with a stopwatch.

(2) Moisture permeability

The moisture permeability of the laminates obtained in Examples B 1 to B5 and Comparative Examples B 1 to B4 was measured based on the moisture permeability testing method (the cup method) according to JIS Z0208-1976. Specifically, 30 g of calcium chloride was placed in a moisture permeable cup; the laminates obtained in Examples and Comparative Examples were left in a measurement environment for two hours or more, and then used as a moisture permeable sheet to cover the moisture permeable cup to achieve airtightness. Then, the increase in the total mass of calcium chloride and the moisture permeable cup in an environment with a wind speed of 0.2 m/s or less after 1 hour was converted into a mass per 1 $m^2$·24 hours of the test piece and measured as the moisture permeability. Note that measurements were separately performed in two environments, one with a temperature of 20°C and a relative humidity of 65%, the other with a temperature of 5°C and a relative humidity of 45%. The moisture permeability at a temperature of 5°C and a relative humidity of 45% was measured only for the laminates obtained in Examples B1 to B3 and Comparative Example B 1.

[Table 2]

Table 2

| | Air Resistance [second/100 cc] | Moisture Permeability at 20°C and 65% RH [g/($m^2$·24h)] | Moisture Permeability at 5°C and 45% RH [g/($m^2$·24h)] |
|---|---|---|---|
| Example B1 | 10000 or greater | 1900 | 500 |
| Example B2 | 7000 | 2300 | 540 |
| Example B3 | 7000 | 2300 | 530 |
| Example B4 | 10000 or greater | 2200 | - |
| Example B5 | 5000 | 2400 | - |
| Comparative Example B1 | 10000 or greater | 1000 | 300 |
| Comparative Example B2 | 10000 or greater | 1700 | - |
| Comparative Example B3 | 500 | 1500 | - |
| Comparative Example B4 | 200 | 2300 | - |

The laminates of Examples B 1 to B5 were evaluated to have high air resistance, meaning low air permeability, and excellent moisture permeability. In particular, the moisture permeability of the laminates of Examples in an environment with a temperature of 5°C and a relative humidity of 45% was 500 g/($m^2$•24 h) or greater (Examples B 1 to B3), and the laminates of Examples B 1 to B5 were evaluated to have excellent moisture permeability in a low temperature and low humidity environment. Meanwhile, the laminates of Comparative Example B 1 to B3 were evaluated to have poor moisture permeability. In addition, in Comparative Example B4 in which only a porous substrate was used, moisture permeability was excellent, but air permeability was high.

Hereinafter, variations of the invention according to the present disclosure will be described.

[Appendix 1] A laminate provided with a porous substrate and a moisture-permeable membrane disposed on at least one side of the porous substrate,
the moisture-permeable membrane being formed of a resin having an attraction value relative to water of -110 kcal/mol or less and a repulsive value relative to water of 35 kcal/mol or greater, the attraction value relative to water being a negative integral value while the repulsive value relative to water being a positive integral value in an energy histogram of a combination of solute and solvent obtained from a process of calculating free energy based on energy representation.

[Appendix 2] The laminate according to Appendix 1, wherein the attraction value is -250 kcal/mol or less.

[Appendix 3] The laminate according to Appendix 1 or 2, wherein the repulsive value is 100 kcal/mol or greater (preferably 140 kcal/mol or greater).

[Appendix 4] The laminate according to any one of Appendices 1 to 3, wherein the resin includes (i) a hydrophilic portion and (ii) one or more hydrophobic portion, the hydrophilic portion being a group represented by Formula (A) and/or a sulfone group, and the one or more hydrophobic portions being selected from the group consisting of: a monovalent hydrocarbon group having two or more carbons in which one or more hydrogen atoms may be substituted by a fluorine atom; a polystyrene backbone; a polyolefin backbone in which one or more hydrogen atoms may be substituted with a fluorine atom; a polyalkadiene backbone; a polyphenylene backbone; a cellulose backbone; and a polyglycerin backbone:

[Chem. 1]

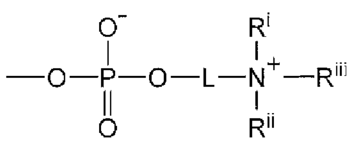

(A)

where in Formula (A) above, $R^i$, $R^{ii}$, and $R^{iii}$ are the same or different and each represents an alkyl group having from 1 to 4 carbons, and L represents a divalent linear hydrocarbon group having from 1 to 4 carbons.

[Appendix 5] The laminate according to Appendix 4, wherein $R^i$, $R^{ii}$, and $R^{iii}$ are a methyl group.

[Appendix 6] The laminate according to Appendix 4 or 5, wherein L is an alkylene group (preferably a dimethylene group).

[Appendix 7] The laminate according to any one of Appendices 4 to 6, wherein the sulfone group is included in the resin as one or more groups selected from the group consisting of: a constituent unit derived from a styrenesulfonic acid; a constituent unit derived from vinylsulfonic acid; an aromatic sulfonic acid group; and a sulfonic acid group bonded to a primary carbon.

[Appendix 8] The laminate according to any one of Appendices 4 to 7, wherein the monovalent hydrocarbon group having two or more carbons is one or more selected from the group consisting of: a monovalent aliphatic hydrocarbon group; a monovalent alicyclic hydrocarbon group; a monovalent aromatic hydrocarbon group; and a monovalent group in which two or more of the groups listed above are bonded.

[Appendix 9] The laminate according to any one of Appendices 4 to 7, wherein the monovalent hydrocarbon group having two or more carbons is a monovalent aliphatic hydrocarbon group (preferably a linear or branched alkyl group, more preferably a linear alkyl group).

[Appendix 10] The laminate according to any one of Appendices 4 to 9, wherein the number of carbons in the monovalent hydrocarbon group having two or more carbons is from 10 to 20.

[Appendix 11] The laminate according to any one of Appendices 4 to 10, wherein the monovalent hydrocarbon group having two or more carbons is a perfluorohydrocarbon group in which all hydrogen atoms are substituted with a fluorine atom.

[Appendix 12] The laminate according to any one of Appendices 4 to 11, wherein the polyolefin backbone is one or more selected from the group consisting of: a polyethylene backbone composed only of ethylene; a polypropylene backbone composed of only propylene; and an ethylene-propylene copolymer backbone composed of ethylene and propylene.

[Appendix 13] The laminate according to any one of Appendices 4 to 12, wherein the polyolefin backbone is a perfluoropolyolefin in which all hydrogen atoms are substituted with a fluorine atom.

[Appendix 14] The laminate according to any one of Appendices 4 to 13, wherein an alkadiene constituting the polyalkadiene backbone is one or more selected from the group consisting of: butadiene; cyclohexadiene; methylcyclohexadiene; cyclooctadiene; methylcyclooctadiene; and phenylcyclooctadiene.

[Appendix 15] The laminate according to any one of Appendices 4 to 14, wherein the resin is one or more resins selected from the group consisting of: a resin having a sulfone group and a polyolefin backbone in which one or more hydrogen atoms may be substituted with a fluorine atom; a resin having a sulfone group and a polyphenylene backbone; a resin having a sulfone group and a polystyrene backbone; a resin having a sulfone group and a polyalkadiene backbone; a resin having a group represented by Formula (A) above and a polyolefin backbone; and a resin having a group represented by Formula (A) above and a monovalent hydrocarbon group having two or more carbons in which one or more hydrogen atoms may be substituted with a fluorine atom.

[Appendix 16] The laminate according to Appendix 15, wherein the resin is soluble or dispersible in room temperature water at a concentration of 0.5 mass% or greater.

[Appendix 17] The laminate according to Appendix 15 or 16, wherein the resin having a sulfone group and a polyolefin backbone in which one or more hydrogen atoms may be substituted with a fluorine atom is a compound having a polyolefin backbone in which one or more hydrogen atoms may be substituted with a fluorine atom as a main chain and an alkoxy group with a sulfone group bonded to the alkoxy end as a side chain.

[Appendix 18] The laminate according to Appendix 17, wherein the alkoxy group is an alkoxy group in which one or more hydrogen atoms are substituted with a fluorine atom.

[Appendix 19] The laminate according to Appendix 15 or 16, wherein the resin having a sulfone group and a polyolefin backbone in which one or more hydrogen atoms may be substituted with a fluorine atom is a compound represented by Formula (I-1) below and/or a compound represented by Formula (I-2) below.

[Chem. 2]

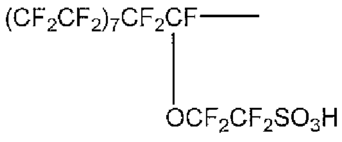
(I-1)

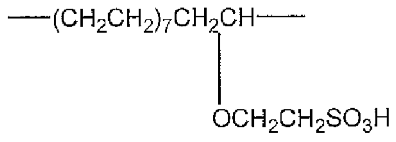
(I-2)

[Appendix 20] The laminate according to any one of Appendices 15 to 19, wherein the resin having a sulfone

group and a polyphenylene backbone is a compound having a polyphenylene backbone as a main chain and an aromatic sulfonic acid group in a side chain.

[Appendix 21] The laminate according to any one of Appendices 15 to 19, wherein the resin having a sulfone group and a polyphenylene backbone is a compound represented by Formula (II-1) below and/or a compound represented by Formula (II-2) below.

[Chem. 3]

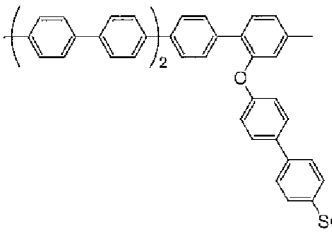

(II-1)

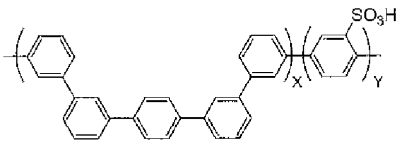

(II-2)

X=1, Y=2

[Appendix 22] The laminate according to any one of Appendices 15 to 21, wherein a resin having a sulfone group and a polystyrene backbone is one or more selected from the group consisting of compounds represented by Formulas (III-1) to (III-3) below.

[Chem. 4]

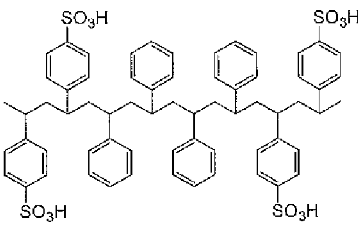

(III-1)

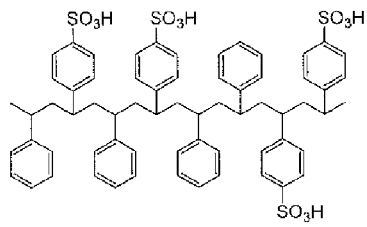

(III-2)

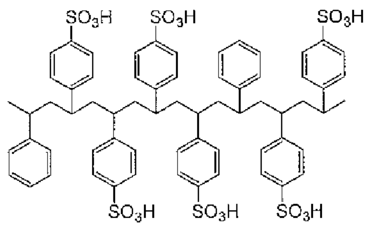

(III-3)

[Appendix 23] The laminate according to any one of Appendices 15 to 22, wherein the resin having a sulfone group and a polyalkadiene backbone is a compound represented by Formula (IV-1) below and/or a compound represented by Formula (IV-2) below.

[Chem. 5]

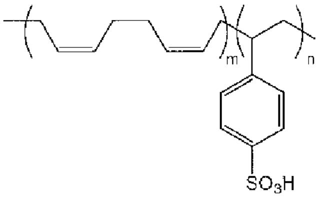

(IV-1)

m=3, n=2

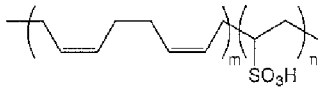

(IV-2)

m=3, n=2

[Appendix 24] The laminate according to any one of Appendices 15 to 23, wherein the resin having a group represented by Formula (A) above and a monovalent hydrocarbon group having two or more carbons in which one or more hydrogen atoms may be substituted with a fluorine atom is a compound represented by Formula (V-1) below.

[Chem. 6]

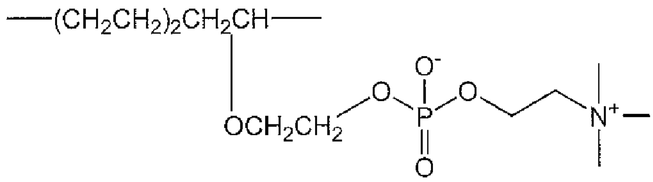

(V-1)

[Appendix 25] The laminate according to any one of Appendices 15 to 23, wherein the resin having a group represented by Formula (A) above and a monovalent hydrocarbon group having two or more carbons in which one or more hydrogen atoms may be substituted with a fluorine atom is a copolymer including a constituent unit represented by Formula (1) below and a constituent unit represented by Formula (2) below.

[Chem. 7]

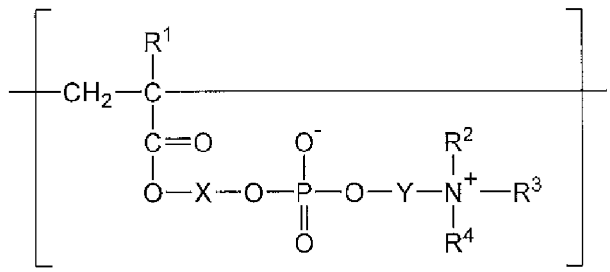

(1)

where in Formula (1), $R^1$ represents a hydrogen atom or an alkyl group having from 1 to 4 carbons; $R^2$, $R^3$, and $R^4$ are the same or different and each represent an alkyl group having from 1 to 4 carbons; X represents a divalent hydrocarbon group having from 1 to 4 carbons; Y represents a divalent linear hydrocarbon group having from 1 to 4 carbons.

[Chem. 8]

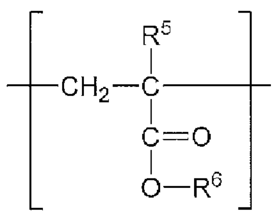

(2)

where in Formula (2), $R^5$ represents a hydrogen atom or an alkyl group having from 1 to 4 carbons, and $R^6$ represents a hydrocarbon group having 2 or more carbons.

[Appendix 26] The laminate according to Appendix 25, wherein $R^1$ is a hydrogen atom or a methyl group (preferably a methyl group).

[Appendix 27] The laminate according to Appendix 25 or 26, wherein $R^2$, $R^3$, and $R^4$ are a methyl group.

[Appendix 28] The laminate according to any one of Appendices 25 to 27, wherein X is a linear or branched alkylene group (preferably a linear alkylene group).

[Appendix 29] The laminate according to any one of Appendices 25 to 28, wherein is an alkylene group (preferably a dimethylene group).

[Appendix 30] The laminate according to any one of Appendices 25 to 29, wherein $R^5$ is a hydrogen atom or a methyl group (preferably a methyl group).

[Appendix 31] The laminate according to any one of Appendices 25 to 30, wherein $R^6$ is a hydrocarbon group having from 4 to 26 carbons (preferably from 14 to 18 carbons).

[Appendix 32] The laminate according to any one of Appendices 25 to 31, wherein $R^6$ is one or more selected from the group consisting of an aliphatic hydrocarbon group, an alicyclic hydrocarbon group, an aromatic hydrocarbon group, and a group in which two or more of the groups listed above are bonded.

[Appendix 33] The laminate according to any one of Appendices 25 to 31, wherein $R^6$ is an aliphatic hydrocarbon group (preferably a linear or branched alkyl group, more preferably a linear alkyl group).

[Appendix 34] The laminate according to any one of Appendices 25 to 33, wherein a molar ratio of the constituent unit represented by Formula (1) above to the constituent unit represented by Formula (2) above (the former/the latter) is from 0.01 to 100.0 (preferably from 0.5 to 5).

[Appendix 35] The laminate according to any one of Appendices 25 to 34, wherein the copolymer is a block copolymer, an alternating copolymer, or a random copolymer (preferably a random copolymer).

[Appendix 36] The laminate according to any one of Appendices 25 to 35, wherein a total number of moles of the constituent unit represented by Formula (1) above and the constituent unit represented by Formula (2) above in the copolymer is 50 mol% or greater (preferably 99 mol% or greater) relative to a total number of moles of constituent units derived from all the monomers constituting the copolymer.

[Appendix 37] The laminate according to any one of Appendices 15 to 23, wherein the resin having a group represented by Formula (A) above and a monovalent hydrocarbon group having two or more carbons in which one or more hydrogen atoms may be substituted with a fluorine atom is a copolymer represented by Formula (VI-1):

[Chem. 9]

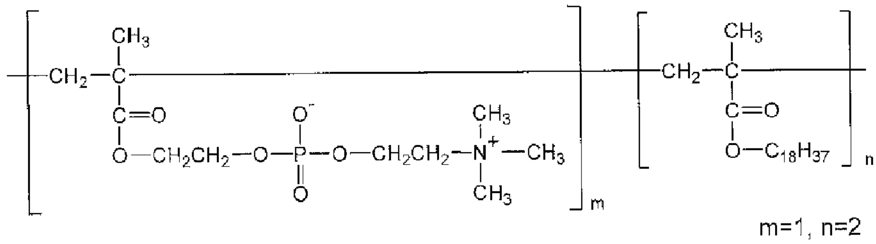

(VI-1)

m=1, n=2

[Appendix 38] The laminate according to any one of Appendices 25 to 37, wherein the copolymer has a weight average molecular weight of from 20000 to 2000000 (preferably from 70000 to 500000.

[Appendix 39] The laminate according to any one of Appendices 1 to 38, wherein the moisture-permeable membrane has a thickness of from 50 to 1000 nm (preferably from 100 to 500 nm).

[Appendix 40] The laminate according to any one of Appendices 1 to 39, wherein a material that forms the porous substrate is one or more selected from the group consisting of a polyolefin-based resin, a cellulose-based resin, a polycarbonate resin, a polyamide resin, a polyimide resin, a polyamide-imide resin, a fluorine-based resin, an inorganic substance such as metal and glass, and paper.

[Appendix 41] The laminate according to any one of Appendices 1 to 39, wherein the material that forms the porous substrate is a polyolefin-based resin (preferably a polypropylene-based resin).

[Appendix 42] The laminate according to any one of Appendices 1 to 41, wherein the porous substrate has a thickness of 5 μm or greater (preferably 10 μm or greater) and 50 μm or less (preferably 30 μm or less).

[Appendix 43] The laminate according to any one of Appendices 1 to 42, wherein a surface tension of the surface of a side of the porous substrate provided with the moisture-permeable membrane is from 35 to 55 dyn/cm (preferably from 37 to 50 dyn/cm).

[Appendix 44] The laminate according to any one of Appendices 1 to 43, wherein a surface tension of an inner part of the porous substrate is less than 35 dyn/cm (preferably 33 dyn/cm or less).

[Appendix 45] The laminate according to any one of Appendices 1 to 44, wherein the laminate has a moisture permeability based on a moisture permeability testing method (the cup method) according to JIS Z0208-1976 under the conditions of a temperature of 20°C, a relative humidity of 65%, and a wind speed of 0.2 m/s or less of 1600 g/($m^2$·24h) or greater [preferably 1800 g/($m^2$·24h) or greater].

[Appendix 46] The laminate according to any one of Appendices 1 to 45, wherein the laminate has a moisture permeability based on a moisture permeability testing method (the cup method) according to JIS Z0208-1976 under the conditions of a temperature of 5°C, a relative humidity of 45%, and a wind speed of 0.2 m/s or less of 300 g/($m^2$·24h) or greater [preferably 500 g/($m^2$·24h) or greater].

[Appendix 47] The laminate according to any one of Appendices 1 to 46, wherein the laminate has an air resistance based on the Gurley method according to JIS P8117-2009 of 3000 seconds/100 cc or greater (preferably 5000 seconds/100 cc or greater).

[Appendix 48] The laminate according to any one of Appendices 1 to 47, wherein the laminate has a rate of decrease in air resistance according to the following water resistance test of 50% or less (preferably 15% or less):

Water resistance test

A test piece of φ7 cm is cut out from the laminate, and the air resistance is measured (initial air resistance). Thereafter, the test piece is immersed in 1 L of room temperature water for 15 minutes, and then naturally dried at room temperature. The immersion and drying counts as one cycle, and the cycle is repeated 50 times on the test piece,

resulting in a test piece after water resistance test. Then, the air resistance of the obtained test piece after water resistance test (air resistance after water resistance test) is measured. Then, the rate of decrease in air resistance is determined in accordance with the following formula. Note that both the initial air resistance above and the air resistance after the water resistance test above are an air resistance based on the Gurley method according to JIS P8117-2009.

$$\text{Rate of decrease in air resistance (\%)} = [(\text{initial air resistance}) - (\text{air resistance after water resistance test})]/(\text{initial air resistance}) \times 100$$

[Appendix 49] The laminate according to any one of Appendices 1 to 48, wherein the laminate has a moisture permeability after the water resistance test based on the moisture permeability testing method (the cup method) according to JIS Z0208-1976 of 1600 g/($m^2 \cdot$24h) or greater [preferably 1800 g/($m^2 \cdot$24h) or greater].

[Appendix 50] A method of calculating an attraction value and a repulsive value relative to a solvent of a target molecule, the method including:

a solvation system creation step of arranging the target molecule in a cubic imaginary space and arranging the solvent molecules around the target molecule in the imaginary space;
a molecular dynamics calculation step of establishing an equilibrium state of the solvation system at room temperature and normal pressure;
a histogram creation step of creating, in the imaginary space, an energy histogram of a combination of solute and solvent in the equilibrium state by free energy calculation based on energy representation; and
an integration step of calculating a negative integral value in the obtained energy histogram as the attraction value relative to solvent of the target molecule and calculating a positive integral value in the obtained energy histogram as the attraction value relative to solvent of the target molecule.

[Appendix 51] The method of calculating an attraction value and a repulsive value according to Appendix 50, wherein in the solvation system creation step, a solvation system is created by centering a target molecule that has undergone multiple steps to align the center of gravity of the target molecule with the origin and arranging the target molecule in a cubic imaginary space in which from 8000 to 12000 water molecules (preferably from 9000 to 10000 water molecules) can be arranged, and randomly arranging solvent molecules in a region inside the imaginary space that is beyond 2Å from the surface of the centered target molecule so that the density is smaller than the room temperature density, the multiple steps that the target molecule that has undergone including:

a target molecule structure modeling step of modeling the three-dimensional molecular structure of the target molecule,
a stable conformation search step, which follows the target molecule structure modeling step, of subjecting the modeled target molecule to a conformation search based on molecular mechanics to determine a stable conformation at the classical-mechanics level,
a structural optimization calculation step, which follows the stable conformation search step, of calculating an optimized structure in vacuum for the target molecule that has undergone the stable conformation search step with the Cartesian coordinates corresponding to the stable conformation at the classical-mechanics level prepared as the input data and based on the density functional theory,
a force field parameter generation step, which follows the structural optimization calculation step, of calculating the partial charge on each atom in the optimized structure of the target molecule obtained in the structural optimization calculation step to generate force field parameters, and
an energy minimization calculation step, which follows the force field parameter generation step, of performing an energy minimization calculation of the target molecule while taking into consideration the force field parameters generated in the force field parameter generation step.

[Appendix 52] The method of calculating an attraction value and a repulsive value according to Appendix 50 or 51, wherein the molecular dynamics calculation step includes:

a first calculation phase, wherein a molecular dynamics calculation is performed at a temperature of over 300 K, a pressure of 1 MPa or greater, and with an addition of an energy constraint of 1 kcal/(mol·$Å^2$) or greater to a minimum energy structure;
a second calculation phase after the first calculation phase, wherein a molecular dynamics calculation is performed while the addition of the energy constraint is reduced until the addition reaches 0 kcal/(mol·$Å^2$);
a third calculation phase after the second calculation phase, wherein a molecular dynamics calculation is performed

while the pressure is reduced until the pressure reaches 1 atm;
a fourth calculation phase after the third calculation phase, wherein a molecular dynamics calculation is performed while the temperature is reduced until the temperature reaches 300 K; and
a fifth calculation phase after the fourth calculation phase, wherein a molecular dynamics calculation is performed at a temperature of 300 K, a pressure of 1 atm, and with the energy constraint lifted, resulting in an equilibrium state of the solvation system at room temperature and normal pressure.

[Appendix 53] The method of calculating an attraction value and a repulsive value according to any one of Appendices 50 to 52, wherein the target molecule is a molecule having a hydrophilic portion and a hydrophobic portion, and the solvent is water.
[Appendix 54] A calculation device for calculating an attraction value and a repulsive value relative to a solvent of a target molecule, the device including:

a solvation system creation portion that arranges the target molecule in a cubic imaginary space and arranges the solvent molecules around the target molecule in the imaginary space;
a molecular dynamics calculation portion that establishes an equilibrium state of the solvation system at room temperature and normal pressure;
a histogram creation portion that creates, in the imaginary space, an energy histogram of a combination of solute and solvent in the equilibrium state by free energy calculation based on energy representation; and
an integration portion that calculates a negative integral value in the obtained energy histogram as the attraction value relative to solvent of the target molecule and calculates a positive integral value in the obtained energy histogram as the attraction value relative to solvent of the target molecule.

Industrial Applicability

The laminate according to an embodiment of the present disclosure has low air permeability and excellent moisture permeability. As such, the laminate according to an embodiment of the present disclosure can be preferably used in, for example, a total heat exchange device, an undergarment, a disposable water-repellent/moisture-permeable material, an application for dehydration without exposure to air or bacteria (a filter for storing aged meat, etc.), a product for improving the environment (air and water quality), and a separation adsorption material. As such, the present disclosure has industrial applicability.

Reference Signs List

1 Laminate
11 Porous substrate
11a One side of porous substrate
12 Moisture-permeable membrane

## Claims

1. A laminate comprising a porous substrate and a moisture-permeable membrane disposed on at least one side of the porous substrate,
the moisture-permeable membrane comprising a resin having an attraction value relative to water of -110 kcal/mol or less and a repulsive value relative to water of 35 kcal/mol or greater, the attraction value relative to water being a negative integral value while the repulsive value relative to water being a positive integral value in an energy histogram of a combination of solute and solvent obtained from a process of calculating free energy based on energy representation.

2. The laminate according to claim 1, wherein the resin comprises (i) a hydrophilic portion, and (ii) one or more hydrophobic portions, the hydrophilic portion comprising a group represented by Formula (A) and/or a sulfone group, the one or more hydrophobic portions being selected from the group consisting of: a monovalent hydrocarbon group having two or more carbons in which one or more hydrogen atoms may be substituted by a fluorine atom; a polystyrene backbone; a polyolefin backbone in which one or more hydrogen atoms may be substituted with a fluorine atom; a polyalkadiene backbone; a polyphenylene backbone; a cellulose backbone; and a polyglycerin backbone:

[Chem. 1]

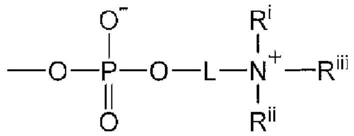

(A)

where in Formula (A), $R^i$, $R^{ii}$, and $R^{iii}$ are the same or different and each represents an alkyl group having from 1 to 4 carbons, and L represents a divalent linear hydrocarbon group having from 1 to 4 carbons.

**3.** The laminate according to claim 2, wherein the sulfone group is included in the resin as one or more groups selected from the group consisting of: a constituent unit derived from a styrenesulfonic acid; a constituent unit derived from vinylsulfonic acid; an aromatic sulfonic acid group; and a sulfonic acid group bonded to a primary carbon.

**4.** The laminate according to claim 2 or 3, wherein the resin comprises one or more resins selected from the group consisting of: a resin having a sulfone group and a polyolefin backbone in which one or more hydrogen atoms may be substituted with a fluorine atom; a resin having a sulfone group and a polyphenylene backbone; a resin having a sulfone group and a polystyrene backbone; a resin having a sulfone group and a polyalkadiene backbone; a resin having a group represented by Formula (A) above and a polyolefin backbone; and a resin having a group represented by Formula (A) above and a monovalent hydrocarbon group having two or more carbons in which one or more hydrogen atoms may be substituted with a fluorine atom.

**5.** A method of calculating an attraction value and a repulsive value relative to a solvent of a target molecule, the method comprising:

a solvation system creation step of arranging the target molecule in a cubic imaginary space and arranging the solvent molecules around the target molecule in the imaginary space;
a molecular dynamics calculation step of establishing an equilibrium state of the solvation system at room temperature and normal pressure;
a histogram creation step of creating, in the imaginary space, an energy histogram of a combination of solute and solvent in the equilibrium state by free energy calculation based on energy representation; and
an integration step of calculating a negative integral value in the obtained energy histogram as the attraction value relative to solvent of the target molecule and calculating a positive integral value in the obtained energy histogram as the attraction value relative to solvent of the target molecule.

**6.** The method of calculating an attraction value and a repulsive value according to claim 5, wherein the molecular dynamics calculation step comprises:

a first calculation phase, wherein a molecular dynamics calculation is performed at a temperature of over 300 K, a pressure of 1 MPa or greater, and with an addition of an energy constraint of 1 kcal(mol.Å$^2$) or greater to a minimum energy structure;
a second calculation phase after the first calculation phase, wherein a molecular dynamics calculation is performed while the addition of the energy constraint is reduced until the addition reaches 0 kcal(mol·Å$^2$);
a third calculation phase after the second calculation phase, wherein a molecular dynamics calculation is performed while the pressure is reduced until the pressure reaches 1 atm;
a fourth calculation phase after the third calculation phase, wherein a molecular dynamics calculation is performed while the temperature is reduced until the temperature reaches 300 K; and
a fifth calculation phase after the fourth calculation phase, wherein a molecular dynamics calculation is performed at a temperature of 300 K, a pressure of 1 atm, and with the energy constraint lifted, resulting in an equilibrium state of the solvation system at room temperature and normal pressure.

**7.** The method of calculating an attraction value and a repulsive value according to claim 5 or 6, wherein the target molecule is a molecule comprising a hydrophilic portion and a hydrophobic portion, and the solvent comprises water.

**8.** A calculation device for calculating an attraction value and a repulsive value relative to a solvent of a target molecule, the device comprising:

a solvation system creation portion configured to arrange the target molecule in a cubic imaginary space and arranges the solvent molecules around the target molecule in the imaginary space;
a molecular dynamics calculation portion configured to establish an equilibrium state of the solvation system at room temperature and normal pressure;
a histogram creation portion configured to create, in the imaginary space, an energy histogram of a combination of solute and solvent in the equilibrium state by free energy calculation based on energy representation; and
an integration portion configured to calculate a negative integral value in the obtained energy histogram as the attraction value relative to solvent of the target molecule and calculates a positive integral value in the obtained energy histogram as the attraction value relative to solvent of the target molecule.

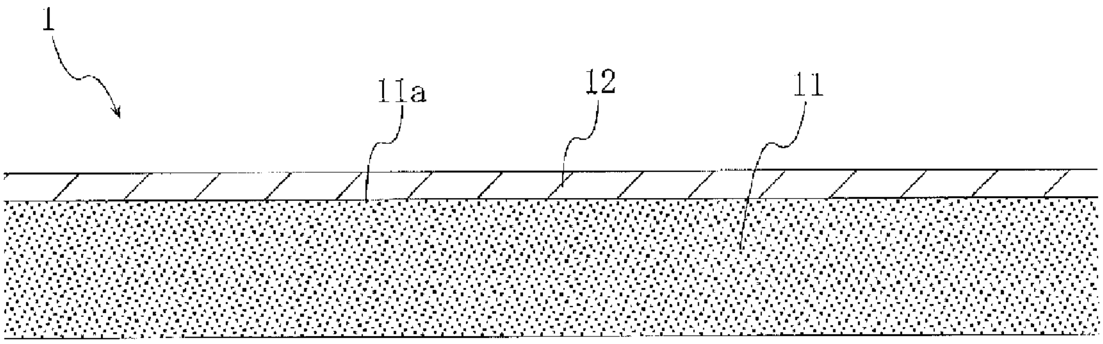


FIG. 1

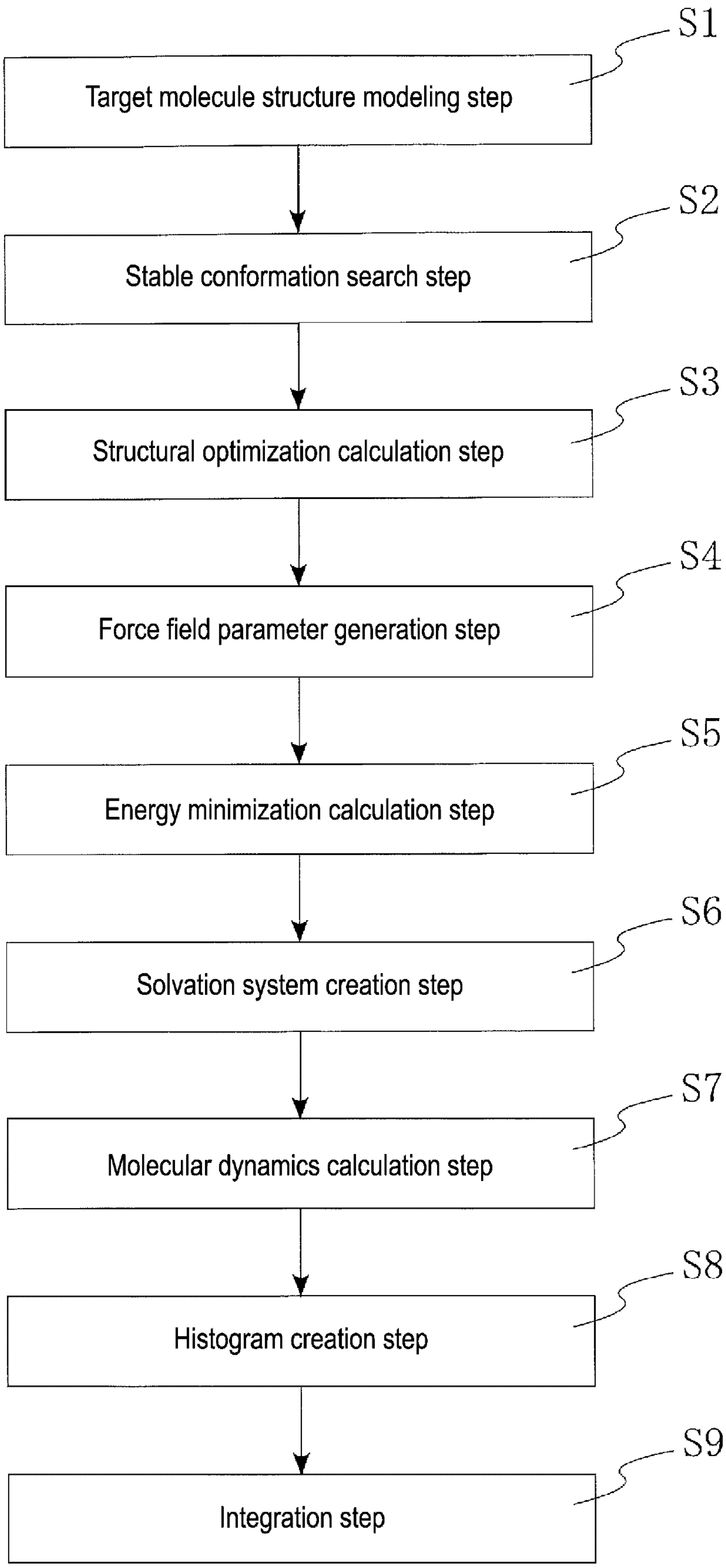
S1
Target molecule structure modeling step
S2
Stable conformation search step
S3
Structural optimization calculation step
S4
Force field parameter generation step
S5
Energy minimization calculation step
S6
Solvation system creation step
S7
Molecular dynamics calculation step
S8
Histogram creation step
S9
Integration step

FIG. 2

## INTERNATIONAL SEARCH REPORT

International application No.
**PCT/JP2021/034104**

**A. CLASSIFICATION OF SUBJECT MATTER**

***B32B 27/00***(2006.01)i; ***B32B 5/18***(2006.01)i; ***F24F 3/14***(2006.01)i; ***F24F 7/08***(2006.01)i; ***F28F 3/00***(2006.01)i
FI: B32B27/00 B; B32B5/18; F28F3/00 301A; F24F7/08 101A; F24F3/14

According to International Patent Classification (IPC) or to both national classification and IPC

**B. FIELDS SEARCHED**

Minimum documentation searched (classification system followed by classification symbols)

B32B1/00-43/00; F24F6/00-7/07; F28F3/00-7/02; F28F99/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2020-509562 A (LG CHEM, LTD.) 26 March 2020 (2020-03-26)<br>paragraphs [0068]-[0073], [0078], [0079] | 1-4<br>5-8 |
| X<br>A | JP 2008-508055 A (KERTZMAN SYSTEMS, INC.) 21 March 2008 (2008-03-21)<br>paragraphs [0023], [0024], [0027], fig. 12 | 1-4<br>5-8 |
| X<br>A | JP 2019-070788 A (CANON INC.) 09 May 2019 (2019-05-09)<br>paragraphs [0085]-[0095], [0121] | 5-8<br>1-4 |
| A | JP 2016-534879 A (ZEHNDER GROUP INTERNATIONAL AG) 10 November 2016 (2016-11-10)<br>claim 7, paragraph [0029] | 1-8 |
| A | JP 2014-055683 A (PANASONIC CORP.) 27 March 2014 (2014-03-27)<br>entire text, all drawings | 1-8 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 November 2021** | **30 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/034104**

**C. DOCUMENTS CONSIDERED TO BE RELEVANT**

| Category* | Citation of document, with indication, where appropriate, of the relevant passages | Relevant to claim No. |
|---|---|---|
| A | JP 2005-050321 A (TORAY INDUSTRIES, INC.) 24 February 2005 (2005-02-24) entire text, all drawings | 1-8 |

Form PCT/ISA/210 (second sheet) (January 2015)

**INTERNATIONAL SEARCH REPORT**
**Information on patent family members**

International application No.
**PCT/JP2021/034104**

| Patent document cited in search report | Publication date (day/month/year) | Patent family member(s) | Publication date (day/month/year) |
|---|---|---|---|
| JP 2020-509562 A | 26 March 2020 | US 2020/0119382 A1<br>paragraphs [0075]-[0079], [0082], [0083]<br>WO 2019/139320 A1<br>EP 3582309 A1<br>KR 10-2019-0085288 A<br>CN 110383553 A | |
| JP 2008-508055 A | 21 March 2008 | US 2006/0021615 A1<br>paragraphs [0040], [0041], [0044], fig. 12<br>GB 2431601 A<br>WO 2006/015224 A2 | |
| JP 2019-070788 A | 09 May 2019 | US 2020/0241377 A1<br>paragraphs [0104]-[0117], [0147]<br>WO 2019/073789 A1<br>EP 3696603 A1<br>CN 111183392 A | |
| JP 2016-534879 A | 10 November 2016 | US 2016/0161131 A1<br>claim 7, paragraph [0032]<br>WO 2015/011544 A1<br>EP 2829834 A1<br>CN 105518409 A<br>KR 10-2016-0034373 A | |
| JP 2014-055683 A | 27 March 2014 | US 2015/0198390 A1<br>entire text, all drawings<br>WO 2014/041746 A1<br>EP 2896924 A1<br>CN 104487795 A | |
| JP 2005-050321 A | 24 February 2005 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020165951 A
- JP 2014055683 A


### Non-patent literature cited in the description


- **N. MATUBAYASI et al.** *J. Chem. Phys.,* 2000, vol. 113, 6070-6081